# EUROPÄISCHE PATENTSCHRIFT

(11) **EP 3 328 352 B1**
(45) Veröffentlichungstag und Bekanntmachung des Hinweises auf die Patenterteilung: **18.12.2019**
(21) Anmeldenummer: 16726601.4
(22) Anmeldetag: 03.06.2016
(51) Int. Cl.: A61K 8/892, A61K 8/58, A61Q 5/10

(54) **AUFHELLVERFAHREN UNTER VERWENDUNG VON SPEZIELLEN SILOXANVERBINDUNGEN**
LIGHTENING PROCESS WITH SPECIFIC SILOXANE COMPOUND
PROCÉDÉ D'ÉCLAIRCISSEMENT AVEC COMPOSÉ SILOXANE SPÉCIFIQUE

(30) Priorität: 28.07.2015 DE 102015214277
(43) Veröffentlichungstag der Anmeldung: 06.06.2018
(73) Patentinhaber: Henkel AG & Co. KGaA, 40589 Düsseldorf (DE)
(72) Erfinder: KERL, Sylvia, 22763 Hamburg (DE); GROSJACQUES, Camille, 20255 Hamburg (DE); KRAUSE, Katharina, 22061 Hamburg (DE)
(86) Internationale Anmeldenummer: PCT/EP2016/062603
(87) Internationale Veröffentlichungsnummer: WO 2017/016719

(56) Entgegenhaltungen:
- WO-A1-2015/086268
- WO-A2-01/41717
- WO-A2-02/087515
- WO-A2-2011/076603
- "Some challenges in modern hair colour formulations", , 1. Januar 1999 (1999-01-01), Seiten 327-340, XP055167563, Gefunden im Internet: URL:http://onlinelibrary.wiley.com/store/1 0.1046/j.1467-2494.1999.186183.x/asset/j.1 467-2494.1999.186183.x.pdf?v=1&t=i5s1f9c7& s=49657cc24769ce37432c6b8610e783051e3ebb41 [gefunden am 2015-02-05]

## Beschreibung

Die vorliegende Erfindung betrifft ein Verfahren zur Aufhellung keratinischer Fasern, bei welchem ein Aufhellmittel (AM) verwendet wird, welches mindestens ein spezielles Dimethylcyclosiloxan sowie mindestens ein spezielles Polydimethylsiloxan, wie in den Ansprüchen beschrieben, enthält. Die Verwendung dieser speziellen Siloxane führt, im Vergleich zu einer Zusammensetzung ohne Siloxane, zu einer verbesserten Pflege bei gleichzeitig verbesserter Aufhellleistung. Stand der Technik ist z.B. WO 2015/086268 A1 (HENKEL AG & CO KGAA [DE]) 18. Juni 2015 (2015-06-18).

Weiterhin betrifft die vorliegende Erfindung eine Verpackungseinheit (Kit-of-parts), umfassend ein kosmetisches Mittel (M1) sowie eine Oxidationsmittelzubereitung (M2), wobei das kosmetische Mittel (M1) und/oder die Oxidationsmittelzubereitung (M2) mindestens ein spezielles Dimethylcyclosiloxan sowie mindestens ein spezielles Polydimethylsiloxan enthalten.

Schließlich betrifft die vorliegende Erfindung die Verwendung einer Kombination mindestens einen speziellen Dimethylcyclosiloxans sowie mindestens eines speziellen Polydimethylsiloxans zur Erhöhung der Pflege keratinischer Fasern bei gleichzeitig verbesserter Aufhellleistung.

Die Veränderung der Form und der Farbe von Haaren stellt einen wichtigen Bereich der modernen Kosmetik dar. Dadurch kann das Erscheinungsbild der Haare sowohl an aktuelle Modeströmungen als auch an die individuellen Wünsche des einzelnen Verbrauchers angepasst werden. Die modische Farbgestaltung von Frisuren oder die Kaschierung von ergrautem oder weißem Haar mit modischen oder natürlichen Farbtönen erfolgt üblicherweise unter Einsatz von farbverändernden Mitteln. Diese Färbeverfahren sollen neben einer hohen Färbeleistung in zusätzlichen Eigenschaften, wie beispielsweise der Steigerung des Haarvolumens, resultieren.

Im Stand der Technik sind verschiedene Färbeverfahren zur Färbung der Haut und/oder von keratinhaltigen Fasern bekannt, bei welchen unterschiedliche farbverändernde kosmetische Mittel eingesetzt werden.

In Färbeverfahren, welche in permanenten, intensiven Färbungen mit entsprechenden Echtheitseigenschaften resultieren, werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehrerer Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muss jedoch üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

In Färbeverfahren, welche in temporäre Färbungen resultieren, werden üblicherweise Färbe- oder Tönungsmittel verwendet, die als färbende Komponente sogenannte Direktzieher enthalten. Hierbei handelt es sich um Farbstoffmoleküle, welche direkt auf das Substrat aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als oxidativen Färbungen, so dass sehr viel früher eine vielfach unerwünschte Nuancenverschiebung oder ein sichtbarer homogener Farbverlust eintritt. Schließlich hat ein weiteres Färbeverfahren große Beachtung gefunden. Bei diesem Verfahren werden Vorstufen des natürlichen Haarfarbstoffes Melanin auf das Substrat, z.B. Haare, aufgebracht; diese bilden dann im Rahmen oxidativer Prozesse im Haar naturanaloge Farbstoffe aus. In solchen Verfahren wird beispielsweise 5,6-Dihydroxyindolin als Farbstoffvorprodukt eingesetzt. Insbesondere bei mehrfacher Anwendung von Mitteln mit 5,6-Dihydroxyindolin ist es möglich, bei Personen mit ergrauten Haaren die natürliche Haarfarbe wiederherzustellen. Die Ausfärbung kann dabei mit Luftsauerstoff als einzigem Oxidationsmittel erfolgen, so dass auf weitere Oxidationsmittel verzichtet werden kann. Bei Personen mit ursprünglich mittelblondem bis braunem Haar kann das 5,6-Dihydroxyindolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden.

Die im Stand der Technik bekannten Färbeverfahren, insbesondere Aufhellverfahren, führen jedoch nicht immer zu der gewünschten hohen Färbeleistung, insbesondere Aufhellleistung, oder weisen zusätzliche gewünschte Eigenschaften, wie beispielsweise eine verbesserte Pflege der Haare während oder nach der Haarfärbung, auf.

Der vorliegenden Erfindung lag daher die Aufgabe zugrunde, ein Verfahren zur Aufhellung von keratinischen Fasern bereitzustellen, welches die Nachteile des Standes der Technik vermeidet bzw. zumindest abschwächt und welches in einer verbesserten Pflege der Haare bei gleichzeitig verbesserter Aufhellleistung resultiert.

Es wurde nun überraschenderweise gefunden, dass der Einsatz von mindestens einem speziellen Dimethylcyclosiloxan sowie mindestens einem Polydimethylsiloxan in Aufhellverfahren zu einer verbesserten Pflege, insbesondere zu einer verbesserten Kämmbarkeit, der keratinischen Fasern bei gleichzeitig verbesserter Aufhellleistung führt.

Ein erster Gegenstand der Erfindung ist daher ein Verfahren zur Aufhellung von keratinischen Fasern, wobei das Verfahren die folgenden Verfahrensschritte in der angegebenen Reihenfolge umfasst:
a) Auftragen eines Aufhellmittels (AM), welches unmittelbar vor der Auftragung aus mindestens einem kosmetischen Mittel (M1) und mindestens einer Oxidationsmittelzubereitung (M2) hergestellt wird, auf die keratinischen Fasern,
b) Belassen des unter Schritt a) hergestellten Aufhellmittels (AM) für eine Zeit von 10 bis 60 Minuten, bevorzugt von 20 bis 45 Minuten, bei Raumtemperatur und/oder bei mindestens 45 °C auf den keratinischen Fasern,
c) Spülen der keratinischen Fasern mit Wasser und/oder einer reinigenden Zusammensetzung für 1 bis 5 Minuten,
wobei das kosmetische Mittel (M1) und/oder die Oxidationsmittelzubereitung (M2) in einem kosmetisch verträglichen Träger
(i) mindestens ein Dimethylcyclosiloxan der Formel (I) 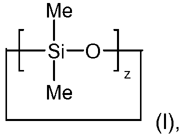 worin
   z für ganze Zahlen von 3 bis 12 steht, und
(ii) mindestens ein Polydimethylsiloxan der Formel (II) 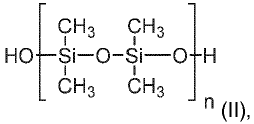 worin n für ganze Zahlen von 1.800 bis 28.000 steht,
enthält.

Unter dem Begriff "keratinische Fasern oder auch Keratinfasern" sind erfindungsgemäß Pelze, Wolle, Federn sowie menschliche Haare zu verstehen. Im Rahmen der vorliegenden Erfindung ist es besonders bevorzugt, wenn das Verfahren zur Aufhellung von menschlichen Haaren verwendet wird.

Weiterhin werden unter dem Begriff "Verfahren zur Aufhellung" im Rahmen der vorliegenden Erfindung Verfahren verstanden, deren Anwendung in einer helleren Färbung der keratinischen Fasern resultiert als vor Durchführung der Aufhellverfahren resultiert. Die Aufhellverfahren der vorliegenden Erfindung können sowohl zur Aufhellung der natürlichen Farbe als auch zur Aufhellung von bereits gefärbten keratinischen Fasern verwendet werden.

Zudem ist unter Raumtemperatur im Rahmen der vorliegenden Erfindung die Umgebungstemperatur zu verstehen, welche ohne Einwirkung von externer Wärme vorherrscht und bevorzugt von 10 bis 39 °C beträgt.

Darüber hinaus ist unter dem Begriff "reinigende Zusammensetzung" erfindungsgemäß eine Zusammensetzung zu verstehen, welche eine Reinigungswirkung aufweist und daher bevorzugt mindestens ein Tensid enthält. Tenside im Sinne der vorliegenden Erfindung sind amphiphile (bifunktionelle) Verbindungen, welche aus mindestens einem hydrophoben und mindestens einem hydrophilen Molekülteil bestehen. Eine Basiseigenschaft von Tensiden ist die orientierte Absorption an Grenzflächen sowie die Aggregation zu Mizellen und die Ausbildung von lyotrophen Phasen.

Weiterhin wird unter dem Begriff "Kämmbarkeit" im Rahmen der vorliegenden Erfindung sowohl die Kämmbarkeit der nassen Faser als auch die Kämmbarkeit der trockenen Faser verstanden.

Darüber hinaus werden unter dem Begriff "Fettalkohole" im Rahmen der vorliegenden Erfindung aliphatische, langkettige, einwertige, primäre Alkohole verstanden, welche unverzweigte Kohlenwasserstoffreste mit 6 bis 30 Kohlenstoffatomen aufweisen. Die Kohlenwasserstoffreste können gesättigt aber auch ein- oder mehrfach ungesättigt sein.

Schließlich werden unter dem Begriff "Fettsäuren" im Rahmen der vorliegenden Erfindung aliphatische Monocarbonsäuren mit unverzweigter Kohlenstoffkette verstanden, welche Kohlenwasserstoffreste mit 6 bis 30 Kohlenstoffatomen aufweisen. Die Kohlenwasserstoffreste können entweder gesättigt oder auch ein- oder mehrfach ungesättigt sein.

Die Angabe der Gesamtmenge in Bezug auf die Komponenten des kosmetischen Mittels (M1) bzw. der Oxidationsmittelzubereitung (M2) bezieht sich vorliegend - sofern nichts anderes angegeben ist - auf die Gesamtmenge an Aktivsubstanz der jeweiligen Komponente.

In Verfahrensschritt a) des erfindungsgemäßen Verfahrens erfolgt die Herstellung eines Aufhellmittels (AM) durch Vermischen eines kosmetischen Mittels (M1) mit einer Oxidationsmittelzubereitung (M2). Es hat sich im Rahmen der vorliegenden Erfindung als vorteilhaft erwiesen, wenn das kosmetische Mittel (M1) mit der Oxidationsmittelzubereitung (M2) in bestimmten Verhältnissen vermischt wird. Bevorzugte erfindungsgemäße Verfahren sind daher dadurch gekennzeichnet, dass in Verfahrensschritt a) das kosmetische Mittel (M1) mit der Oxidationsmittelzubereitung (M2) im Gewichtsverhältnis 3 : 1 bis 1 : 3, vorzugsweise von 2 : 1 bis 1 : 2, insbesondere 1 : 2, vermischt wird. Die Auftragung des durch Vermischen hergestellten Aufhellmittels (AM) erfolgt üblicherweise mit der Hand durch den Anwender. Bevorzugt wird dabei persönliche Schutzkleidung getragen, insbesondere geeignete Schutzhandschuhe, beispielsweise aus Kunststoff oder Latex zur einmaligen Benutzung (Einweghandschuhe) sowie gegebenenfalls eine Schürze. Es ist aber auch möglich, das Aufhellmittel (AM) mit einer Applikationshilfe auf die keratinischen Fasern aufzutragen.

In Verfahrensschritt b) wird das in Schritt a) hergestellte Aufhellmittel (AM) bei Raumtemperatur und/oder bei mindestens 45 °C auf den keratinischen Fasern belassen. Hierbei ist es möglich, das Aufhellmittel (AM) zunächst einige Zeit bei Raumtemperatur einwirken zu lassen und anschließend die Temperatur auf mindestens 45 °C zu erhöhen. Durch Erhöhung der Temperatur, beispielsweise durch externe Wärmezufuhr mittels einer Wärmehaube, kann die Aufhellleistung des erfindungsgemäßen Verfahrens verstärkt werden.

Nach Beendigung der Einwirkdauer in Verfahrensschritt b) wird das verbliebene Aufhellmittel (AM) aus den keratinischen Fasern mit Hilfe einer Reinigungszubereitung, welche bevorzugt mindestens ein kationisches und/oder anionisches und/oder nichtionisches Tensid enthält, und/oder Wasser ausgewaschen (Verfahrensschritt c)). Gegebenenfalls wird dieser Verfahrensschritt c) mindestens ein weiteres Mal wiederholt.

Im Rahmen der vorliegenden Erfindung kann es weiterhin vorteilhaft sein, nach dem Verfahrensschritt c) in einem nachfolgenden Verfahrensschritt d) ein Nachbehandlungsmittel auf die keratinischen Fasern aufzutragen. Dieses Nachbehandlungsmittel kann beispielsweise ein Konditioniermittel, welches mindestens eine konditionierende Verbindung aus der Gruppe von kationischen Polymeren, Siliconderivaten und Ölen enthält, sein. Es ist daher im Rahmen der vorliegenden Erfindung bevorzugt, wenn nach Verfahrensschritt c) in einem Verfahrensschritt d) ein Nachbehandlungsmittel auf die keratinischen Fasern aufgetragen und diese nach einer Zeit von 1 bis 10 Minuten ausgespült werden. Erfindungsgemäß bevorzugt werden die Verfahrensschritte a) bis d) in der zuvor angeführten Reihenfolge mit einem Zeitabstand zwischen den einzelnen Verfahrensschritten von jeweils 0 bis 60 Minuten, vorzugsweise von jeweils 0 bis 40 Minuten, insbesondere von jeweils 0 bis 30 Minuten, durchgeführt.

Das in dem erfindungsgemäßen Verfahren eingesetzte Aufhellmittel (AM) enthält mindestens ein spezielles Dimethylcyclosiloxan der Formel (I).

Es hat sich erfindungsgemäß als vorteilhaft erwiesen, wenn Dimethylcyclosiloxane mit einer bestimmten Ringgröße eingesetzt werden. Bevorzugt erfindungsgemäße Verfahren sind daher dadurch gekennzeichnet, dass in der Formel (I) z für ganze Zahlen von 3 bis 10, vorzugsweise von 4 bis 8, bevorzugt von 4 bis 6, insbesondere für 5, steht.

Besonders gute Ergebnisse im Hinblick auf die Aufhellleistung sowie die Pflegeleistung werden erhalten, wenn ein Dimethylcyclosiloxan der Formel (la) eingesetzt wird. Es ist daher im Rahmen der vorliegenden Erfindung besonders bevorzugt, wenn das mindestens eine Dimethylcyclosiloxan (i) in dem kosmetischen Mittel (M1) und/oder in der Oxidationsmittelzubereitung (M2) die Formel (la) 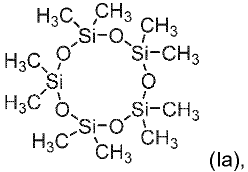 aufweist.

Bevorzugt wird das mindestens eine Dimethylcyclosiloxan (i) in dem kosmetischen Mittel (M1) und/oder der Oxidationsmittelzubereitung (M2) in bestimmten Mengenbereichen eingesetzt. Erfindungsgemäß vorteilhafte Verfahren sind daher dadurch gekennzeichnet, dass das kosmetische Mittel (M1) und/oder die Oxidationsmittelzubereitung (M2) das mindestens eine Dimethylcyclosiloxan (i) der Formel (I) und/oder der Formel (la) in einer Gesamtmenge von 0,1 bis 17 Gew.-%, vorzugsweise von 0,5 bis 8,5 Gew.-%, bevorzugt von 1,0 bis 6,0 Gew.-%, insbesondere von 1,2 bis 4,5 Gew.-%, bezogen auf das Gesamtgewicht des kosmetischen Mittels (M1) und/oder der Oxidationsmittelzubereitung (M2), enthält. Der Einsatz des mindestens einen Dimethylcyclosiloxans (i) der Formel (I) und/oder der Formel (la) in den zuvor genannten Mengenbereichen führt zu einer besonders hohen Pflege- und/oder Aufhellleistung des erfindungsgemäßen Verfahrens.

Weiterhin enthält das in dem erfindungsgemäßen Verfahren eingesetzte Aufhellmittel (AM) mindestens ein Polydimethylsiloxan der Formel (II).

Es ist im Rahmen der vorliegenden Erfindung vorteilhaft, Polydimethylsiloxane (ii) mit einem bestimmten Molekulargewicht einzusetzen. Bevorzugte Ausführungsformen der vorliegenden Erfindung zeichnen sich daher dadurch aus, dass das mindestens eine Polydimethylsiloxan (ii) der Formel (II) in dem kosmetischen Mittel (M1) und/oder der Oxidationsmittelzubereitung (M2) ein mittleres Molekulargewicht M_{w} von 140.000 bis 2.000.000 Da, vorzugsweise von 150.000 bis 1.900.000 Da, bevorzugt von 160.000 bis 1.800.000 Da, insbesondere von 170.000 bis 1.700.000 Da, aufweist. Das mittlere Molekulargewicht M_{w} kann beispielsweise durch Gelpermeationschromatographie (GPC) bestimmt werden (Liu X. M. et. al.; "Comparative Studies of Poly(DimethylSiloxanes) Using Automated GPC-MALDI-TOF MS and On-Line GPC-ESI-TOF MS"; Am. Soc. Mass. Spectrom., 2003, 14, Seiten 195 bis 202).

Darüber hinaus hat es sich im Hinblick auf die verbesserte Pflege- und/oder Aufhellleistung des erfindungsgemäßen Verfahrens als vorteilhaft erwiesen, wenn das Polydimethylsiloxan (ii) der Formel (II) eine bestimmte Viskosität aufweist. Bevorzugte erfindungsgemäße Verfahren sind daher dadurch gekennzeichnet, dass das mindestens eine Polydimethylsiloxan (ii) der Formel (II) in dem kosmetischen Mittel (M1) und/oder die Oxidationsmittelzubereitung (M2) eine Viskosität bei 25 °C von 100.000 bis 100.000.000 mPa*s, vorzugsweise von 200.000 bis 100.000.000 mPa*s, bevorzugt von 300.000 bis 100.000.000 mPa*s, insbesondere von 400.000 bis 100.000.000 mPa*s, aufweist. Die Bestimmung der Viskosität des Polydimethylsiloxans (ii) kann beispielsweise wie in der Offenlegungsschrift US 2014/0004073 A1 beschrieben durchgeführt werden.

Erfindungsgemäß bevorzugt wird das mindestens eine Polydimethylsiloxan (ii) in bestimmten Mengenbereichen eingesetzt. Es ist daher im Rahmen der vorliegenden Erfindung vorteilhaft, wenn das kosmetische Mittel (M1) und/oder der Oxidationsmittelzubereitung (M2) das mindestens eine Polydimethylsiloxan (ii) der Formel (II) in einer Gesamtmenge von 0,005 bis 3,0 Gew.-%, vorzugsweise von 0,05 bis 1,5 Gew.-%, bevorzugt von 0,1 bis 1,2 Gew.-%, insbesondere von 0,2 bis 1,0 Gew.-%, bezogen auf das Gesamtgewicht des kosmetischen Mittels (M1) und/oder der Oxidationsmittelzubereitung (M2), enthält. Der Einsatz der zuvor genannten Mengenbereiche des mindestens einen Polydimethylsiloxans (ii) der Formel (II) in Kombination mit dem mindestens einen Dimethylcyclosiloxan der Formel (I) und/oder der Formel (la) führt zu einer besonders hohen Pflege- und/oder Aufhellleistung des erfindungsgemäßen Verfahrens.

Besonders gute Ergebnisse im Hinblick auf die Pflege- und/oder Aufhellleistung werden weiterhin erhalten, wenn das kosmetische Mittel (M1) und/oder die Oxidationsmittelzubereitung (M2) das mindestens eine Dimethylcyclosiloxan (i) der Formel (I) und/oder der Formel (la) sowie das mindestens eine Polydimethylsiloxan (ii) in bestimmten Gewichtsverhältnissen enthalten. Bevorzugte erfindungsgemäße Verfahren sind daher dadurch gekennzeichnet, dass das Gewichtsverhältnis des mindestens einen Dimethylcyclosiloxans (i) der Formel (I) und/oder der Formel (la) zu dem mindestens einen Polydimethylsiloxan (ii) der Formel (II) in dem kosmetischen Mittel (M1) und/oder der Oxidationsmittelzubereitung (M2) von 20 : 1 bis 1 : 1, vorzugsweise von 15 : 1 bis 2 : 1, bevorzugt von 10 : 1 bis 3 : 1, insbesondere von 6 : 1 bis 4 : 1, beträgt. Das zuvor angegebene Gewichtsverhältnis bezieht sich hierbei auf die jeweiligen Gesamtmengen des mindestens einen Dimethylcyclosiloxans (i) der Formel (I) und/oder der Formel (la) sowie des mindestens einen Polydimethylsiloxans (ii) der Formel (II).

In besonders bevorzugte Verfahren der vorliegenden Erfindung enthält das kosmetische Mittel (M1) und/oder die Oxidationsmittelzubereitung (M2) ein Dimethylcyclosiloxan der Formel (la) sowie ein spezielles Polydimethylsiloxan (ii) der Formel (II). Es ist daher erfindungsgemäß besonders bevorzugt, wenn das kosmetische Mittel (M1) und/oder die Oxidationsmittelzubereitung (M2)
(i) mindestens ein Dimethylcyclosiloxan der Formel (Ia) 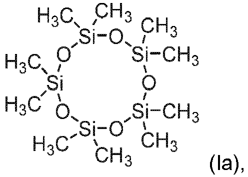 und
(ii) mindestens ein Polydimethylsiloxan der Formel (II) 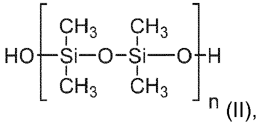 worin
   n für 1.800 bis 28.000 steht, enthält.

Im Hinblick auf die Pflege- und Aufhellleistung hat es sich als besonders vorteilhaft erwiesen, wenn in dem kosmetischen Mittel (M1) und/oder der Oxidationsmittelzubereitung (M2) ein Dimethylcyclosiloxan der Formel (la) sowie ein spezielles Polydimethylsiloxan der Formel (II) in bestimmten Mengen und Gewichtsverhältnissen enthalten sind. Besonders bevorzugte erfindungsgemäße Verfahren sind daher dadurch gekennzeichnet, dass das kosmetische Mittel (M1) und/oder die Oxidationsmittelzubereitung (M2) - bezogen auf das Gesamtgewicht des kosmetischen Mittels (M1) und/oder der Oxidationsmittelzubereitung (M2) -
(i) mindestens ein Dimethylcyclosiloxan der Formel (la) in einer Gesamtmenge von 1,2 bis 4,5 Gew.-% 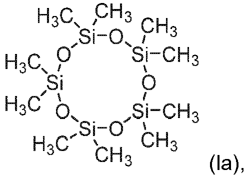
(ii) mindestens ein Polydimethylsiloxan der Formel (II) in einer Gesamtmenge von 0,2 bis 1,0 Gew.-% 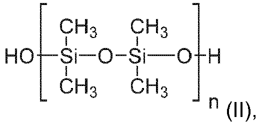 worin
   n für 1.800 bis 28.000 steht, enthält,
wobei das Gewichtsverhältnis des mindestens einen Dimethylcyclosiloxans (i) der Formel (la) zu dem mindestens einen Polydimethylsiloxan (ii) der Formel (II) in dem kosmetischen Mittel (M1) und/oder der Oxidationsmittelzubereitung (M2) von 6 : 1 bis 4 : 1 beträgt. Der Einsatz der zuvor angeführten Siloxane in dem kosmetischen Mittel (M1) und/oder der Oxidationsmittelzubereitung (M2) führt zu einer verbesserten Pflege- und/oder Aufhellleistung im Vergleich zu Aufhellverfahren, in welchen die zuvor genannte Kombination nicht eingesetzt wird.

Das in dem erfindungsgemäßen Verfahren verwendete kosmetische Mittel (M1) sowie die Oxidationsmittelzubereitung (M2) enthalten die zuvor angeführten Siloxane der Formeln (I) und/oder (la) sowie (II) in einem kosmetischen Träger. Erfindungsgemäß bevorzugt ist der kosmetische Träger wässrig, alkoholisch oder wässrig-alkoholisch. Im Rahmen der vorliegenden Erfindung können beispielsweise Cremes, Emulsionen, Gele oder tensidhaltige schäumende Lösungen, wie beispielsweise Shampoos, Schaumaerosole oder andere Zubereitungen, welche für die Anwendung auf dem Haar geeignet sind, eingesetzt werden.

Ein wässriger Träger enthält im Sinne der Erfindung mindestens 30 Gew.-%, insbesondere mindestens 50 Gew.-%, Wasser, bezogen auf das Gesamtgewicht des kosmetischen Mittels (M1) oder der Oxidationsmittelzubereitung (M2).

Unter wässrig-alkoholischen Trägern sind im Sinne der vorliegenden Erfindung wasserhaltige Zusammensetzungen, enthaltend einen C₁-C₄-Alkohol in einer Gesamtmenge von 3 bis 90 Gew.-%, bezogen auf das Gesamtgewicht des kosmetischen Mittels (M1) oder der Oxidationsmittelzubereitung (M2), insbesondere Ethanol bzw. Isopropanol, zu verstehen.

Das erfindungsgemäß verwendete kosmetische Mittel (M1) sowie die Oxidationsmittelzubereitung (M2) können zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel, wobei das Lösungsmittel in einer Gesamtmenge von 0,1 bis 30 Gew.-%, vorzugsweise von 1 bis 20 Gew.-%, insbesondere von 2 bis 10 Gew.-%, bezogen auf das Gesamtgewicht des kosmetischen Mittels (M1) oder der Oxidationsmittelzubereitung (M2), enthalten ist. Das erfindungsgemäß verwendete kosmetische Mittel (M1) kann zusätzlich eine farbgebende, insbesondere aufhellende, Verbindung enthalten. Im Rahmen der vorliegenden Erfindung ist es bevorzugt, wenn das kosmetische Mittel (M1) zusätzlich mindestens eine farbgebende Verbindung, ausgewählt aus der Gruppe von Oxidationsfarbstoffvorprodukten, direktziehenden Farbstoffen sowie deren Mischungen, enthält.

In einer bevorzugten Ausführungsform enthält das kosmetische Mittel (M1) mindestens ein Oxidationsfarbstoffvorprodukt.

Oxidationsfarbstoffvorprodukte können aufgrund ihres Reaktionsverhaltens in zwei Kategorien eingeteilt werden, sogenannte Entwicklerkomponenten und Kupplerkomponenten. Entwicklerkomponenten können mit sich selbst den eigentlichen Farbstoff ausbilden. Sie können daher als alleinige Verbindungen im erfindungsgemäß verwendeten kosmetischen Mittel (M1) enthalten sein. In einer bevorzugten Ausführungsform enthalten die kosmetischen Mittel (M1) daher mindestens ein Oxidationsfarbstoffvorprodukt vom Entwicklertyp. Es kann im Rahmen der vorliegenden Erfindung jedoch auch vorgesehen sein, dass die kosmetischen Mittel (M1) mindestens ein Oxidationsfarbstoffvorprodukt vom Kupplertyp enthalten. Besonders gute Ergebnisse in Bezug auf die Färbung keratinischer Fasern werden erhalten, wenn die kosmetischen Mittel (M1) mindestens ein Oxidationsfarbstoffvorprodukt vom Entwicklertyp und mindestens ein Oxidationsfarbstoffvorprodukt vom Kupplertyp enthalten.

Die Entwickler- und Kupplerkomponenten werden üblicherweise in freier Form eingesetzt. Bei Substanzen mit Aminogruppen kann es jedoch bevorzugt sein, deren Salzform, insbesondere in Form der Hydrochloride und Hydrobromide oder der Sulfate, einzusetzen.

Erfindungsgemäß sind kosmetische Mittel (M1) bevorzugt, welche die Entwickler- und/oder Kupplerkomponenten jeweils in einer Gesamtmenge von 0,001 bis 10 Gew.-%, vorzugsweise von 0,01 bis 8 Gew.-%, bevorzugt von 0,1 bis 5 Gew.-%, insbesondere von 0,5 bis 3 Gew.-%, bezogen auf das Gesamtgewicht des kosmetischen Mittels (M1), enthalten.

In einer weiteren bevorzugten Ausführungsform ist das erfindungsgemäß verwendete kosmetische Mittel (M1) daher dadurch gekennzeichnet, dass es ein Oxidationsfarbstoffvorprodukt vom Entwickler und/oder Kupplertyp in einer Gesamtmenge von 0,001 bis 10 Gew.-%, vorzugsweise von 0,01 bis 8 Gew.-%, bevorzugt von 0,1 bis 5 Gew.-%, insbesondere von 0,5 bis 3 Gew.-%, bezogen auf das Gesamtgewicht des kosmetischen Mittels (M1), enthält.

Geeignete Oxidationsfarbstoffvorprodukte vom Entwicklertyp sind beispielsweise p-Phenylendiamin und dessen Derivate. Bevorzugte p-Phenylendiamine werden ausgewählt aus einer oder mehrerer Verbindungen der Gruppe, welche gebildet wird aus p-Phenylendiamin, p-Toluylendiamin, 2-Chlor-p-phenylendiamin, 2,3-Dimethyl-p-phenylendiamin, 2,6-Dimethyl-p-phenylendiamin, N,N-Bis-(2-hydroxyethyl)-p-phenylen-diamin, 2-(2-Hydroxyethyl)-p-phenylendiamin, 2-(1,2-Dihydroxyethyl)-p-phenylendiamin, N-(2-Hydroxypropyl)-p-phenylendiamin, N-(4'-Aminophenyl)-p-phenylendiamin, 2-Methoxymethyl-p-phenylendiamin, N-Phenyl-p-phenylendiamin, 2-(2-Hydroxyethyloxy)-p-phenylendiamin und N-(4-Amino-3-methyl-phenyl)-N-[3-(1H-imidazol-1-yl)propyl]amin sowie ihren physiologisch verträglichen Salzen.

Es kann erfindungsgemäß weiterhin bevorzugt sein, als Entwicklerkomponente Verbindungen einzusetzen, welche mindestens zwei aromatische Kerne enthalten, die mit Amino- und/oder Hydroxylgruppen substituiert sind. Bevorzugte zweikernige Entwicklerkomponenten werden ausgewählt aus N,N'-Bis-(2-hydroxyethyl)-N,N'-bis-(4'-aminophenyl)-1,3-diaminopropan-2-ol, N,N'-Bis-(4-aminophenyl)-1,4-diazacycloheptan, Bis-(2-hydroxy-5-aminophenyl)methan sowie deren physiologisch verträglichen Salzen.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind p-Aminophenol, N-Methyl-p-Aminophenol, 4-Amino-3-methylphenol, 4-Amino-2-aminomethyl-phenol, 4-Amino-2-(1,2-dihydroxyethyl)phenol, 4-Amino-2-(diethylaminomethyl)phenol sowie deren physiologisch verträglichen Salze.

Ferner kann die Entwicklerkomponente ausgewählt sein aus o-Aminophenol und dessen Derivaten, bevorzugt aus 2-Amino-4-methylphenol, 2-Amino-5-methylphenol, 2-Amino-4-chlorphenol und/oder deren physiologisch verträglichen Salzen.

Weiterhin kann die Entwicklerkomponente ausgewählt sein aus heterocyclischen Entwicklerkomponenten, wie Pyrimidin-Derivaten, Pyrazol-Derivaten, Pyrazolopyrimidin-Derivaten bzw. ihren physiologisch verträglichen Salzen. Bevorzugte Pyrimidin-Derivate sind 2,4,5,6-Tetraaminopyrimidin, 4-Hydroxy-2,5,6-triaminopyrimidin und deren physiologisch verträgliche Salze. Ein bevorzugtes Pyrazol-Derivat ist 4,5-Diamino-1-(2-hydroxyethyl)pyrazol sowie dessen physiologisch verträglichen Salze. Als Pyrazolopyrimidine sind insbesondere Pyrazolo[1,5-a]pyrimidine bevorzugt.

Bevorzugte Oxidationsfarbstoffvorprodukte vom Entwicklertyp sind ausgewählt aus der Gruppe, welche gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-amino-methylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin oder den physiologisch verträglichen Salzen dieser Verbindungen. Besonders bevorzugte Entwicklerkomponenten sind p-Toluylendiamin, 2-(2-Hydroxyethyl)-p-phenylendiamin, 2-Methoxymethyl-p-phenylendiamin, N-(4-Amino-3-methylphenyl)-N-[3-(1 H-imidazol-1-yl)-propyl]amin, und/oder 4,5-Diamino-1-(2-hydroxyethyl)pyrazol sowie deren physiologisch verträglichen Salze.

Gemäß einer weiteren bevorzugten Ausführungsform der vorliegenden Erfindung enthält das kosmetische Mittel (M1) als Oxidationsfarbstoffvorprodukt neben mindestens einer Entwicklerkomponente weiterhin zusätzlich mindestens eine Kupplerkomponente. Als Kuppler-komponenten werden in der Regel m-Phenylendiaminderivate, Naphthole, Resorcin und Resorcinderivate, Pyrazolone und m-Aminophenolderivate verwendet.

Erfindungsgemäß bevorzugte Kupplerkomponenten sind ausgewählt aus
(A) m-Aminophenol und dessen Derivaten, insbesondere 3-Aminophenol, 5-Amino-2-methylphenol, 3-Amino-2-chlor-6-methylphenol, 5-Amino-4-chlor-2-methylphenol, 5-(2'-Hydroxyethyl)-amino-2-methylphenol und 2,4-Dichlor-3-aminophenol,
(B) o-Aminophenol und dessen Derivaten, wie 2-Amino-5-ethylphenol,
(C) m-Diaminobenzol und dessen Derivaten, wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 2,6-Bis-(2'-hydroxyethylamino)-1-methylbenzol, 2-({3-[(2-Hydroxyethyl)amino]-4-methoxy-5-methylphenyl}amino)ethanol und 2-({3-[(2-Hydroxyethyl)amino]-2-methoxy-5-methylphenyl}-amino)ethanol,
(D) o-Diaminobenzol und dessen Derivaten,
(E) Di- beziehungsweise Trihydroxybenzolderivaten, insbesondere Resorcin, 2-Chlorresorcin, 4-Chlorresorcin, 2-Methylresorcin und 1,2,4-Trihydroxybenzol,
(F) Pyridinderivaten, insbesondere 3-Amino-2-methylamino-6-methoxypyridin, 2,6-Diaminopyridin, 2,6-Dihydroxy-3,4-dimethylpyridin, 2-Amino-3-hydroxypyridin und 3,5-Diamino-2,6-dimethoxy-pyridin,
(G) Naphthalinderivaten, wie 1-Naphthol und 2-Methyl-1-naphthol,
(H) Morpholinderivaten, wie 6-Hydroxybenzomorpholin,
(I) Chinoxalinderivaten,
(J) Pyrazolderivaten, wie 1-Phenyl-3-methylpyrazol-5-on,
(K) Indolderivaten, wie 6-Hydroxyindol,
(L) Pyrimidinderivaten oder
(M) Methylendioxybenzolderivaten, wie 1-(2'-Hydroxyethyl)-amino-3,4-methylendioxybenzol
sowie deren physiologisch verträglichen Salze.

Erfindungsgemäß bevorzugte Kupplerkomponenten sind ausgewählt aus der Gruppe, welche gebildet wird aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-di-aminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder den physiologisch verträglichen Salzen der vorgenannten Verbindungen.

Erfindungsgemäß besonders bevorzugte Kupplerkomponenten sind Resorcin, 2-Methylresorcin, 4-Chlorresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin, 2,6-Dihydroxy-3,4-dimethylpyridin, 1,5-Dihydroxynaphthalin, 2,7-Dihydroxynaphthalin und 1-Naphthol sowie deren physiologisch verträgliche Salze.

In einer besonders bevorzugten Ausführungsform der vorliegenden Erfindung sind die erfindungsgemäß verwendeten kosmetischen Mittel (M1) dadurch gekennzeichnet, dass sie als Oxidationsfarbstoffvorprodukt mindestens eine Entwicklerkomponente, ausgewählt aus der Gruppe von p-Phenylendiamin, p-Toluylendiamin, N,N-Bis-(2-hydroxyethyl)amino-p-phenylendiamin, 1,3-Bis-[(2-hydroxyethyl-4'-aminophenyl)amino]-propan-2-ol, 1,10-Bis-(2',5'-diaminophenyl)-1,4,7,10-tetraoxadecan, 4-Aminophenol, 4- Amino-3-methylphenol, Bis-(5- amino-2-hydroxyphenyl)methan, 2,4,5,6- Tetraaminopyrimidin, 2-Hydroxy-4,5,6-triaminopyrimidin, 4,5-Diamino-1-(2-hydroxyethyl)-pyrazol, deren physiologisch verträglichen Salze sowie deren Mischungen, und mindestens eine Kupplerkomponente, ausgewählt aus der Gruppe von Resorcin, 2-Methylresorcin, 5-Methyl-resorcin, 2,5-Dimethylresorcin, 4-Chlorresorcin, Resorcinmonomethylether, 5-Aminophenol, 5-Amino-2-methylphenol, 5-(2-Hydroxyethyl)amino-2-methylphenol, 3- Amino-4-chlor-2-methylphenol, 3-Amino-2-chlor-6-methylphenol, 3-Amino-2,4-Dichlorphenol, 2,4-Diaminophenoxyethanol, 2-Amino-4-(2'-hydroxyethyl)amino-anisolsulfat, 1,3-Bis-(2,4-diaminophenoxy)propan, 2-Amino-3-hydroxypyridin, 2-Methylamino-3-amino-6-methoxypyridin, 2,6-Dihydroxy-3,4-dimethylpyridin, 3,5-Diamino-2,6-dimethoxypyridin, 1-Naphthol, 2-Methyl-1-naphthol, 1,5-Dihydroxynaphthalin, 2,7-Dihydroxynaphthalin, 1-Phenyl-3-methylpyrazol-5-on, 2,6-Bis-[(2'-hydroxyethyl)amino]-toluol, 4-Hydroxyindol, 6-Hydroxyindol, 6-Hydroxybenzomorpholin, deren physiologisch verträglichen Salze sowie deren Mischungen, enthalten. Um eine ausgewogene und subtile Nuancenausbildung zu erhalten, kann es im Rahmen der vorliegenden Erfindung auch vorgesehen sein, dass die kosmetischen Mittel (M1) zusätzlich mindestens einen direktziehenden Farbstoff enthalten. Bei direktziehenden Farbstoffen handelt sich um Farbstoffe, welche direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Direktziehende Farbstoffe können in anionische, kationische und nichtionische direktziehende Farbstoffe unterteilt werden.

Bevorzugte anionische direktziehende Farbstoffe sind die unter den Bezeichnungen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52 und Tetrabromphenolblau bekannten Verbindungen. Bevorzugte kationische direktziehende Farbstoffe sind kationische Triphenylmethanfarbstoffe, wie Basic Blue 7, Basic Blue 26, Basic Violet 2 und Basic Violet 14 sowie aromatische Systeme, welche mit einer quaternären Stickstoffgruppe substituiert sind, wie Basic Yellow 57, Basic Red 76, Basic Blue 99, Basic Brown 16 und Basic Brown 17 und HC Blue 16, sowie Basic Yellow 87, Basic Orange 31 und Basic Red 51. Bevorzugte nichtionische direktziehende Farbstoffe sind HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Walnuss, Faulbaumrinde, Salbei, Blauholz, Krappwurzel, Catechu und Alkannawurzel enthalten sind.

Bevorzugt enthält das kosmetische Mittel (M1) die direktziehenden Farbstoffe in einer Gesamtmenge von 0,001 bis 10 Gew.-%, vorzugsweise von 0,01 bis 8 Gew.-%, bevorzugt von 0,1 bis 5 Gew.-%, insbesondere von 0,5 bis 3 Gew.-%, bezogen auf das Gesamtgewicht des kosmetischen Mittels (M1).

Die erfindungsgemäß verwendeten kosmetischen Mittel (M1) können weitere Wirk- und Zusatzstoffe enthalten. Es ist daher im Rahmen der vorliegenden Erfindung bevorzugt, wenn das kosmetische Mittel (M1) zusätzlich mindestens eine weitere Verbindung, ausgewählt aus der Gruppe von (i) Verdickungsmitteln; (ii) linearen oder verzweigten, gesättigten oder ungesättigten Alkoholen mit 8 bis 20 Kohlenstoffatomen; (iii) Tensiden, insbesondere amphoteren Tensiden; (iv) Alkalisierungsmitteln; (v) Ölen; sowie (vi) deren Mischungen, enthält.

Bevorzugt werden die erfindungsgemäß verwendeten kosmetischen Mittel (M1) als fließfähige Zubereitungen formuliert. Dabei sollten die kosmetischen Mittel (M1) so formuliert werden, dass diese nach Vermischen mit der Oxidationsmittelzubereitung (M2) in Aufhellmitteln (AM) resultieren, welche sich einerseits gut am Anwendungsort auftragen und verteilen lassen, andererseits jedoch ausreichend viskos sind, so dass sie während der Einwirkzeit am Wirkort verbleiben und nicht verlaufen.

Es hat sich daher erfindungsgemäß als vorteilhaft erwiesen, wenn die kosmetischen Mittel (M1) mindestens ein Verdickungsmittel aus der Gruppe von (i) anionischen, synthetischen Polymeren; (ii) kationischen, synthetischen Polymeren; (iii) natürlich vorkommenden Verdickungsmitteln, wie nichtionischen Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektinen, Alginaten, Stärke-Fraktionen und Derivaten, wie Amylose, Amylopektin und Dextrinen, sowie Cellulosederivaten, wie beispielsweise Methylcellulose, Carboxyalkylcellulosen und Hydroxyalkylcellulosen; (iv) nichtionischen, synthetischen Polymeren, wie Polyvinylalkohol oder Polyvinylpyrrolidinon; (v) anorganischen Verdickungsmitteln, insbesondere Schichtsilikaten wie beispielsweise Bentonit, besonders Smektite, wie Montmorillonit oder Hectorit; sowie (vi) deren Mischungen, in einer Gesamtmenge von 0,0005 bis 5,0 Gew.-%, vorzugsweise von 0,001 bis 3,0 Gew.-%, bevorzugt von 0,005 bis 1,0 Gew.-%, insbesondere von 0,008 bis 0,01 Gew.-%, bezogen auf das Gesamtgewicht des kosmetischen Mittels (M1), enthalten.

Es hat sich im Rahmen der vorliegenden Erfindung als besonders vorteilhaft erwiesen, wenn als Verdickungsmittel mindestens ein natürlich vorkommendes Verdickungsmittel, insbesondere Xanthangum sowie dessen Salze, in einer Gesamtmenge von 0,0005 bis 5,0 Gew.-%, vorzugsweise von 0,001 bis 1,0 Gew.-%, bevorzugt von 0,005 bis 0,5 Gew.-%, insbesondere von 0,01 bis 0,1 Gew.-%, bezogen auf das Gesamtgewicht des kosmetischen Mittels (M1), enthalten ist.

Im Rahmen der vorliegenden Erfindung kann es bevorzugt sein, wenn der lineare oder verzweigte, gesättigte oder ungesättigte Alkohol mit 8 bis 20 Kohlenstoffatomen ausgewählt ist aus der Gruppe von Myristylalkohol (1-Tetradecanol), Stearylalkohol (1-Octadecanol), Cetearylalkohol, 2-Octyldodecanol, Arachylalkohol (Eicosan-1-ol), Gadoleylalkohol ((9Z)-Eicos-9-en-1-ol), Arachidonalkohol ((5Z,8Z,11Z,14Z)-Eicosa-5,8,11,14-tetraen-1-ol), vorzugsweise 2-Octyldodecanol und/oder Cetearylalkohol, und in einer Gesamtmenge von 1,0 bis 35 Gew.-%, vorzugsweise von 5,0 bis 30 Gew.-%, bevorzugt von 10 bis 25 Gew.-%, insbesondere von 12 bis 20 Gew.-%, bezogen auf das Gesamtgewicht des kosmetischen Mittels (M1), enthalten ist.

Bevorzugt können die erfindungsgemäß verwendeten kosmetischen Mittel (M1) weiterhin mindestens einen Partialester aus einem Polyol mit 2 bis 6 Kohlenstoffatomen und linearen gesättigten Carbonsäuren mit 12 bis 30, insbesondere 14 bis 22 Kohlenstoffatomen, wobei die Partialester hydroxyliert sein können, in einer Gesamtmenge von 0,5 bis 10 Gew.-%, insbesondere von 3,0 bis 8,0 Gew.-%, bezogen auf das Gesamtgewicht des kosmetischen Mittels (M1), enthalten. Solche Partialester sind insbesondere die Mono- und Diester von Glycerin oder die Monoester von Propylenglycol oder die Mono- und Diester von Ethylenglycol oder die Mono-, Di-, Tri- und Tetraester von Pentaerythrit jeweils mit linearen gesättigten C₁₂ - C₃₀-Carbonsäuren, welche hydroxyliert sein können, insbesondere diejenigen mit Palmitin- und Stearinsäure, die Sorbitanmono-, -di- oder -triester von linearen gesättigten C₁₂ - C₃₀-Carbonsäuren, welche hydroxyliert sein können, insbesondere diejenigen von Myristinsäure, Palmitinsäure, Stearinsäure oder von Mischungen dieser Fettsäuren und die Methylglucosemono- und -diester von linearen gesättigten C₁₂ - C₃₀-Carbonsäuren, welche hydroxyliert sein können.

Im Rahmen der vorliegenden Erfindung kann es vorgesehen sein, dass die erfindungsgemäß verwendeten kosmetischen Mittel (M1) mindestens einen Polyolpartialester, ausgewählt aus Glycerinmonostearat, Glycerinmonopalmitat, Glycerindistearat, Glycerindipalmitat, Ethylenglycolmonostearat, Ethylenglycolmonopalmitat, Ethylenglycoldistearat, Ethylenglycoldipalmitat, sowie Mischungen hiervon, insbesondere Mischungen aus Glycerinmonostearat, Glycerinmonopalmitat, Glycerindistearat und Glycerindipalmitat in einer Gesamtmenge von 0,5 bis 10 Gew.-%, insbesondere von 3,0 bis 8,0 Gew.-%, bezogen auf das Gesamtgewicht des kosmetischen Mittels (M1), enthalten. Der Einsatz der zuvor angeführten Alkohole, Partialester und Polypartialester in den erfindungsgemäß verwendeten kosmetischen Mitteln (M1) kann insbesondere dann bevorzugt sein, wenn die erfindungsgemäß verwendeten kosmetischen Mittel (M1) in Form einer ÖI-in-Wasser-Emulsion vorliegen.

Weiterhin kann es erfindungsgemäß vorgesehen sein, dass die kosmetischen Mittel (M1) gemäß der Erfindung mindestens ein Tensid enthalten. Tenside im Sinne der vorliegenden Erfindung sind amphiphile (bifunktionelle) Verbindungen, welche aus mindestens einem hydrophoben und mindestens einem hydrophilen Molekülteil bestehen. Eine Basiseigenschaft von Tensiden und Emulgatoren ist die orientierte Absorption an Grenzflächen sowie die Aggregation zu Mizellen und die Ausbildung von lyotrophen Phasen.

Gemäß einer bevorzugten Ausführungsform der vorliegenden Erfindung enthalten die erfindungsgemäß verwendeten kosmetischen Mittel (M1) mindestens ein amphoteres Tensid in einer Gesamtmenge von 0,1 bis 5,0 Gew.-%, insbesondere von 0,2 bis 2,0 Gew.-%, bezogen auf das Gesamtgewicht des kosmetischen Mittels. Als amphotere bzw. zwitterionische Tenside werden solche oberflächenaktiven Verbindungen bezeichnet, welche mindestens eine quartäre Ammoniumgruppe und mindestens eine - COO⁽⁻⁾- oder -SO3⁽⁻⁾-Gruppe aufweisen.

Als amphotere Tenside sind im Rahmen der vorliegenden Erfindung die nachfolgend genannten Verbindungen besonders bevorzugt:
- Alkylbetaine mit 8 bis 20 Kohlenstoffatomen in der Alkylgruppe,
- Amidopropylbetaine mit 8 bis 20 Kohlenstoffatomen in der Acylgruppe,
- Sulfobetaine mit 8 bis 20 Kohlenstoffatomen in der Acylgruppe, und
- Amphoacetate oder Amphodiacetate mit 8 bis 20 Kohlenstoffatomen in der Acylgruppe.

In einer besonders bevorzugten Ausführungsform enthalten die kosmetischen Mittel (M1) als Tensid mindestens ein amphoteres Tensid, ausgewählt aus Amidopropylbetainen mit 9 bis 13 Kohlenstoffatomen in der Acylgruppe, in einer Gesamtmenge von 0,1 bis 5,0 Gew.-%, insbesondere von 0,2 bis 2,0 Gew.-%, bezogen auf das Gesamtgewicht des kosmetischen Mittels (M1).

Weiterhin kann es vorgesehen sein, dass die erfindungsgemäß verwendeten kosmetischen Mittel (M1) mindestens ein ethoxyliertes nichtionisches Tensid in einer Gesamtmenge von 0,5 bis 6,0 Gew.-%, insbesondere von 1,0 bis 4,0 Gew.-%, bezogen auf das Gesamtgewicht des kosmetischen Mittels (M1), enthalten. Dabei hat es sich als besonders vorteilhaft erwiesen, wenn das ethoxylierte nichtionische Tensid einen HLB-Wert oberhalb von 10, vorzugsweise oberhalb von 13 aufweist. Hierzu ist es erforderlich, dass das nichtionische Tensid einen ausreichend hohen Ethoxylierungsgrad besitzt. In diesem Zusammenhang enthält das kosmetische Mittel (M1) daher als ethoxyliertes nichtionisches Tensid mindestens ein ethoxyliertes Tensid mit mindestens 12 Ethylenoxideinheiten. Neben den entsprechend ethoxylierten Fettalkoholen, insbesondere Laurylalkohol, Myristylalkohol, Cetylalkohol, Stearylalkohol, Arachylalkohol und Behenylalkohol, sind erfindungsgemäß insbesondere die Anlagerungsprodukte von 20 bis 60 Mol Ethylenoxid an Rizinusöl und gehärtetes Rizinusöl besonders geeignet. Das mindestens eine ethoxylierte nichtionische Tensid ist bevorzugt ausgewählt aus Tensiden mit der INCI-Bezeichnung Ceteth-12, Steareth-12, Ceteareth-12, Ceteth-20, Steareth-20, Ceteareth-20, Ceteth-30, Steareth-30, Ceteareth-30, Oleth-30, Ceteareth-50, PEG-40 Hydrogenated Castor Oil und PEG-60 Hydrogenated Castor Oil sowie Mischungen dieser Substanzen, besonders bevorzugt ausgewählt aus Ceteth-20, Steareth-20, Ceteareth-20, Ceteth-30, Steareth-30 und Ceteareth-30. Kosmetische Mittel (M1) im Rahmen der vorliegenden Erfindung weisen in der Regel einen basischen pH-Wert, insbesondere zwischen pH 8,0 und pH 12, auf. Diese pH-Werte sind erforderlich, um eine Öffnung der äußeren Schuppenschicht (Cuticula) zu gewährleisten und eine Penetration der Oxidationsfarbstoffvorprodukte und/oder des Oxidationsmittels in das Haar zu ermöglichen.

Die Einstellung des zuvor genannten pH-Wertes kann bevorzugt unter Verwendung eines Alkalisierungsmittels erfolgen. Im Rahmen der vorliegenden Erfindung ist das Alkalisierungsmittel ausgewählt aus der Gruppe von (i) anorganischen Alkalisierungsmitteln; (ii) organischen Alkalisierungsmitteln; sowie (iii) deren Mischungen, und in einer Gesamtmenge von 1,5 bis 9,5 Gew.-%, vorzugsweise von 2,5 bis 8,5 Gew.-%, bevorzugt von 3,0 bis 8,0 Gew.-%, insbesondere von 3,5 bis 7,5 Gew.-%, bezogen auf das Gesamtgewicht des kosmetischen Mittels (M1), enthalten.

Bevorzugte anorganische Alkalisierungsmittel sind ausgewählt aus der Gruppe, welche gebildet wird aus Ammoniak bzw. Ammoniumhydroxid, also wässrigen Lösungen von Ammoniak, Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat sowie Mischungen hiervon. Ammoniak bzw. Ammoniumhydroxid ist ein besonders bevorzugtes Alkalisierungsmittel. Besonders bevorzugt ist Ammoniak in einer Gesamtmenge von 0,1 bis 20 Gew.-%, vorzugsweise von 0,5 bis 10 Gew.-%, insbesondere von 1,0 bis 7,0 Gew.-%, bezogen auf das Gesamtgewicht des kosmetischen Mittels (M1), enthalten.

Bevorzugte organische Alkalisierungsmittel sind ausgewählt aus mindestens einem Alkanolamin. Erfindungsgemäß bevorzugte Alkanolamine sind ausgewählt aus Alkanolaminen aus primären, sekundären oder tertiären Aminen mit einem C₂-C₆-Alkylgrundkörper, welcher mindestens eine Hydroxylgruppe trägt. Besonders bevorzugte Alkanolamine werden aus der Gruppe ausgewählt, welche gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethyl-ethanolamin, Triethanolamin, Diethanolamin und Triisopropanolamin. Erfindungsgemäß ganz besonders bevorzugte Alkanolamine werden ausgewählt aus der Gruppe von 2-Aminoethan-1-ol (Monoethanolamin), 2-Amino-2-methyl-propan-1-ol, 2-Amino-2-methyl-propan-1,3-diol und Triethanolamin. Besonders bevorzugte kosmetische Mittel (M1) enthalten eine Mischung aus Monoethanolamin und 2-Amino-2-methylpropan-1ol. Bevorzugt ist das mindestens eine Alkanolamin in einer Gesamtmenge von 0,05 bis 15 Gew.-%, vorzugsweise von 0,5 bis 10 Gew.-%, insbesondere von 3,5 bis 7,5 Gew.-%, bezogen auf das Gesamtgewicht des kosmetischen Mittels (M1), enthalten.

Weitere erfindungsgemäß bevorzugte organische Alkalisierungsmittel sind ausgewählt aus basischen Aminosäuren, besonders bevorzugt ausgewählt aus der Gruppe, welche gebildet wird aus L-Arginin, D-Arginin, D/L-Arginin, L-Lysin, D-Lysin, D/L-Lysin sowie Mischungen hiervon. Erfindungsgemäß besonders bevorzugte basische Aminosäuren sind ausgewählt aus L-Arginin, D-Arginin und D/L-Arginin. Bevorzugte kosmetische Mittel (M1) enthalten mindestens ein von Alkanolaminen und Ammoniak verschiedenes Alkalisierungsmittel in einer Gesamtmenge von 0,05 bis 5,0 Gew.-%, insbesondere von 0,5 bis 3,0 Gew.-%, bezogen auf das Gesamtgewicht des kosmetischen Mittels (M1). Erfindungsgemäß bevorzugt ist das Alkalisierungsmittel ausgewählt aus der Gruppe von Natriumhydroxid, Kaliumhydroxid, Ammoniak, Monoethanolamin und 2-Amino-2-methylpropan, insbesondere Monoethanolamin, und in einer Gesamtmenge von 1,5 bis 9,5 Gew.-%, vorzugsweise von 2,5 bis 8,5 Gew.-%, bevorzugt von 3,0 bis 8,0 Gew.-%, insbesondere von 3,5 bis 7,5 Gew.-%, bezogen auf das Gesamtgewicht des kosmetischen Mittels (M1), enthalten.

In einer besonders bevorzugten Ausführungsform enthalten die kosmetischen Mittel (M1) als Alkalisierungsmittel eine Mischung aus mindestens zwei voneinander verschiedenen Alkanolaminen, insbesondere von Monoethanolamin und 2-Amino-2-methylpropan-1-ol, in einer Gesamtmenge von 0,05 bis 15 Gew.-%, vorzugsweise von 0,5 bis 10 Gew.-%, insbesondere von 3,5 bis 7,5 Gew.-%, bezogen auf das Gesamtgewicht des kosmetischen Mittels (M1).

Bevorzugt beträgt der pH-Wert der kosmetischen Mittel (M1), gemessen bei 22°C, 8 bis 13, vorzugsweise 9,5 bis 12, bevorzugt 10 bis 11,5, insbesondere 10,5 bis 11.

Im Rahmen der vorliegenden Erfindung kann es weiterhin bevorzugt sein, wenn die kosmetischen Mittel (M1) mindestens ein Öl, ausgewählt aus der Gruppe von Sonnenblumenöl, Maisöl, Sojaöl, Kürbiskernöl, Traubenkernöl, Sesamöl, Haselnussöl, Aprikosenkernöl, Macadamianussöl, Araraöl, Rizinusöl, Avocadoöl sowie deren Mischungen, in einer Gesamtmenge von 0,1 bis 10 Gew.-%, bevorzugt von 0,2 bis 5,0 Gew.-%, insbesondere von 0,5 bis 2,0 Gew.-%, bezogen auf das Gesamtgewicht des kosmetischen Mittels (M1), enthalten. Durch den Einsatz mindestens eines zuvor genannten Öls kann der Pflegeeffekt der Kombination von speziellen Siloxanen weitergehend gesteigert werden. Besonders bevorzugt enthalten die kosmetischen Mittel (M1) Traubenkernöl in einer Gesamtmenge von 0,1 bis 10 Gew.-%, bevorzugt von 0,2 bis 5,0 Gew.-%, insbesondere von 0,5 bis 2,0 Gew.-%, bezogen auf das Gesamtgewicht des kosmetischen Mittels (M1).

In nachfolgenden Tabellen sind besonders bevorzugte Ausführungsformen AF 1 bis AF 28 der erfindungsgemäß verwendeten kosmetischen Mittel (M1) aufgeführt (alle Angaben in Gew.-%, sofern nichts anderes angegeben ist):

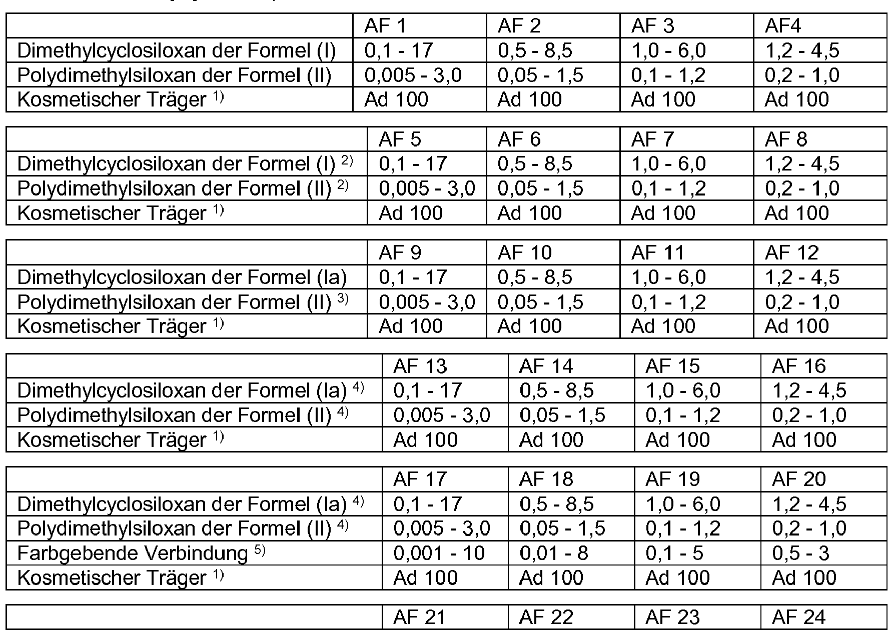

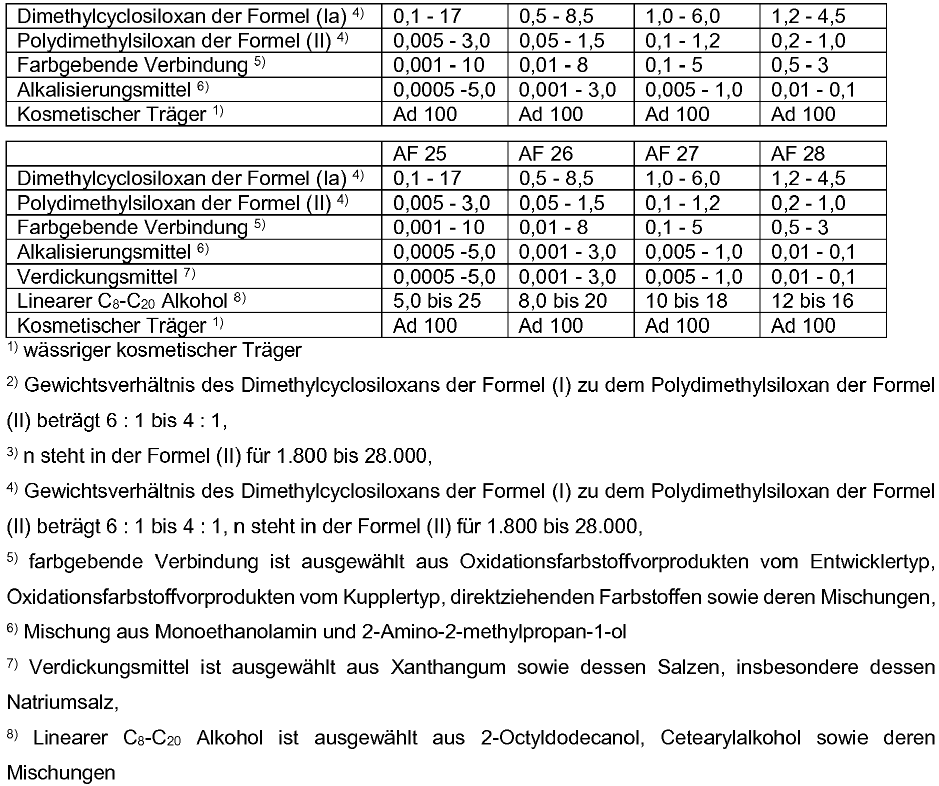

Die erfindungsgemäß verwendete Oxidationsmittelzubereitung (M2) enthält bevorzugt mindestens ein Oxidationsmittel. Die Oxidationsmittel im Rahmen der vorliegenden Erfindung sind von Luftsauerstoff verschieden. Als Oxidationsmittel können Wasserstoffperoxid sowie dessen feste Anlagerungsprodukte an organische und anorganische Verbindungen eingesetzt werden. Als feste Anlagerungsprodukte kommen erfindungsgemäß insbesondere die Anlagerungsprodukte an Harnstoff, Melamin, Polyvinylpyrrolidinon sowie Natriumborat in Frage. Wasserstoffperoxid und/oder eines seiner festen Anlagerungsprodukte an organische oder anorganische Verbindungen sind als Oxidationsmittel besonders bevorzugt. Erfindungsgemäß enthält die Oxidationsmittelzubereitung (M2) mindestens ein Oxidationsmittel aus der Gruppe von Wasserstoffperoxid und dessen Anlagerungsprodukten an Harnstoff, Melamin und Natriumborat, insbesondere Wasserstoffperoxid, in einer Gesamtmenge von 0,5 bis 25 Gew.-%, vorzugsweise von 4,0 bis 20 Gew.-%, bevorzugt von 8,0 bis 13 Gew.-%, insbesondere von 9,0 bis 12 Gew.-%, bezogen auf das Gesamtgewicht der Oxidationsmittelzubereitung (M2). Die zuvor angegebene Gesamtmenge bezieht sich hierbei auf 100%iges Wasserstoffperoxid bzw. auf 100%ige Anlagerungsprodukte von Wasserstoffperoxid.

Eine besonders bevorzugte Ausführungsform der vorliegenden Erfindung ist somit dadurch gekennzeichnet, dass als Oxidationsmittel Wasserstoffperoxid in einer Gesamtmenge von 0,5 bis 25 Gew.-%, vorzugsweise von 4,0 bis 20 Gew.-%, bevorzugt von 6,0 bis 15 Gew.-%, besonders bevorzugt von 8,0 bis 13 Gew.-%, insbesondere von 9,0 bis 12 Gew.-%, bezogen auf das Gesamtgewicht der Oxidationsmittelzubereitung (M2), enthalten ist. Die Berechnung der Gesamtmenge bezieht sich hierbei auf 100 %-iges H₂O₂.

Die Oxidationsmittelzubereitungen (M2) können weiterhin Wasser in einer Gesamtmenge von 40 bis 98 Gew.-%, insbesondere von 65 bis 85 Gew.-%, bezogen auf das Gesamtgewicht der Oxidationsmittelzubereitung (M2), enthalten.

Darüber hinaus können die Oxidationsmittelzubereitungen (M2) weitere Wirk- und Hilfsstoffe enthalten. Bevorzugt enthält die Oxidationsmittelzubereitung (M2) zusätzlich mindestens eine weitere Verbindung, ausgewählt aus der Gruppe von (i) linearen oder verzweigten, gesättigten oder ungesättigten Alkoholen mit 8 bis 20 Kohlenstoffatomen, (ii) nichtionischen Tensiden, (iii) Estern aus Carbonsäuren mit 10 bis 20 Kohlenstoffatomen und linearen oder verzweigten Alkoholen mit 1 bis 5 Kohlenstoffatomen, (iv) Säuren, sowie (v) deren Mischungen.

In diesem Zusammenhang hat es sich als vorteilhaft erwiesen, wenn der lineare oder verzweigte, gesättigte oder ungesättigte Alkohol mit 8 bis 20 Kohlenstoffatomen ausgewählt ist aus der Gruppe von Myristylalkohol (1-Tetradecanol), Stearylalkohol (1-Octadecanol), Cetearylalkohol, 2-Octyldodecanol, Arachylalkohol (Eicosan-1-ol), Gadoleylalkohol ((9Z)-Eicos-9-en-1-ol), Arachidonalkohol ((5Z,8Z,11Z,14Z)-Eicosa-5,8,11,14-tetraen-1-ol), vorzugsweise 2-Octyldodecanol und/oder Cetearylalkohol, und in einer Gesamtmenge von 0,1 bis 10 Gew.-%, vorzugsweise von 0,5 bis 5,0 Gew.-%, insbesondere von 1,0 bis 4,0 Gew.-%, bezogen auf das Gesamtgewicht der Oxidationsmittelzubereitung (M2), enthalten ist. Bevorzugt sind insbesondere Cetylalkohol, Stearylalkohol, Arachidylalkohol, Behenylalkohol und Lanolinalkohol oder Gemische dieser Alkohole, wie sie bei der großtechnischen Hydrierung von pflanzlichen und tierischen Fettsäuren erhältlich sind, sowie Mischungen dieser Alkanole. Besonders bevorzugt ist die Mischung Cetearylalkohol. Erfindungsgemäß ebenfalls vorteilhaft ist der zusätzliche Einsatz mindestens eines nichtionischen Tensids, insbesondere eines ethoxylierten nichtionischen Tensids. Das ethoxylierte nichtionische Tensid ist bevorzugt ausgewählt aus Tensiden mit der INCI-Bezeichnung Ceteth-12, Steareth-12, Ceteareth-12, Ceteth-20, Steareth-20, Ceteareth-20, Ceteth-30, Steareth-30, Ceteareth-30, Oleth-30, Ceteareth-50, PEG-40 Hydrogenated Castor Oil und PEG-60 Hydrogenated Castor Oil sowie Mischungen dieser Substanzen, besonders bevorzugt ausgewählt aus Ceteth-20, Steareth-20, Ceteareth-20, Ceteth-30, Steareth-30 und Ceteareth-30, und in einer Gesamtmenge von 0,1 bis 10 Gew.-%, vorzugsweise von 0,5 bis 5,0 Gew.-%, insbesondere von 1,0 bis 4,0 Gew.-%, bezogen auf das Gesamtgewicht der Oxidationsmittelzubereitung (M2), enthalten.

In diesem Zusammenhang kann es weiterhin bevorzugt sein, wenn der Ester aus einer Carbonsäure mit 10 bis 20 Kohlenstoffatomen und einem linearen oder verzweigten Alkohol mit 1 bis 5 Kohlenstoffatomen ausgewählt ist aus Isopropylmyristat und in einer Gesamtmenge von 3,0 bis 25 Gew.-%, vorzugsweise von 5,0 bis 20 Gew.-%, insbesondere von 8,0 bis 15 Gew.-%, bezogen auf das Gesamtgewicht der Oxidationsmittelzubereitung (M2), enthalten ist.

Die erfindungsgemäßen Oxidationsmittelzubereitungen (M2) können weiterhin mindestens eine Säure enthalten. Bevorzugte Säuren sind ausgewählt aus Dipicolinsäure, Genusssäuren, wie beispielsweise Zitronensäure, Essigsäure, Apfelsäure, Milchsäure und Weinsäure, verdünnten Mineralsäuren, wie Salzsäure, Phosphorsäure, Pyrophosphorsäure und Schwefelsäure, sowie Mischungen hiervon.

Die Oxidationsmittelzubereitungen (M2) weisen bevorzugt einen pH-Wert im Bereich von 2 bis 5, insbesondere von 3 bis 4, auf.

In nachfolgenden Tabellen sind besonders bevorzugte Ausführungsformen AF 29 bis AF 60 der erfindungsgemäß verwendeten Oxidationsmittelzubereitungen (M2) aufgeführt (alle Angaben in Gew.-%, sofern nichts anderes angegeben ist):

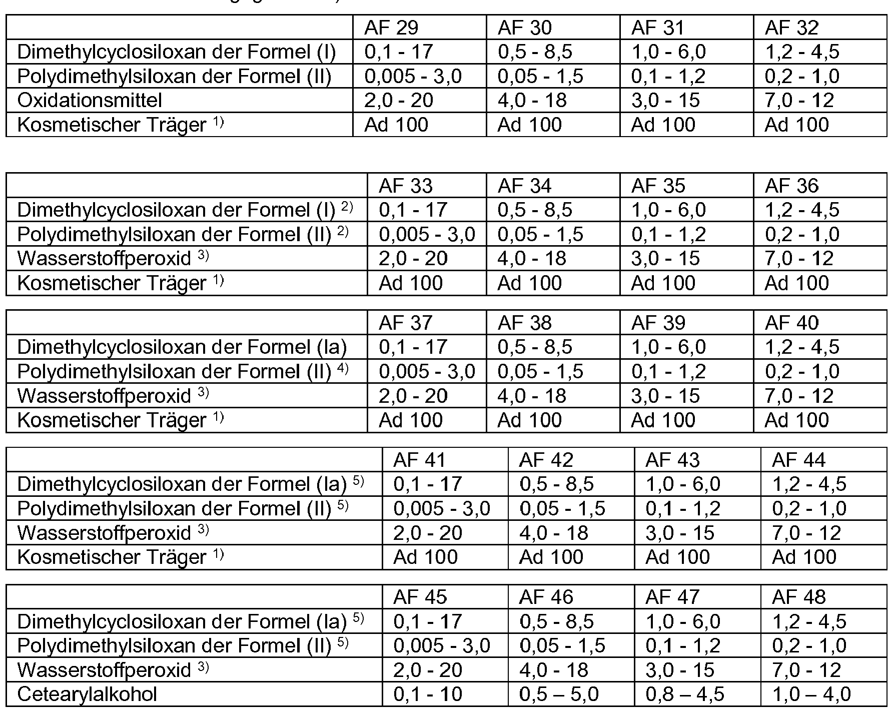

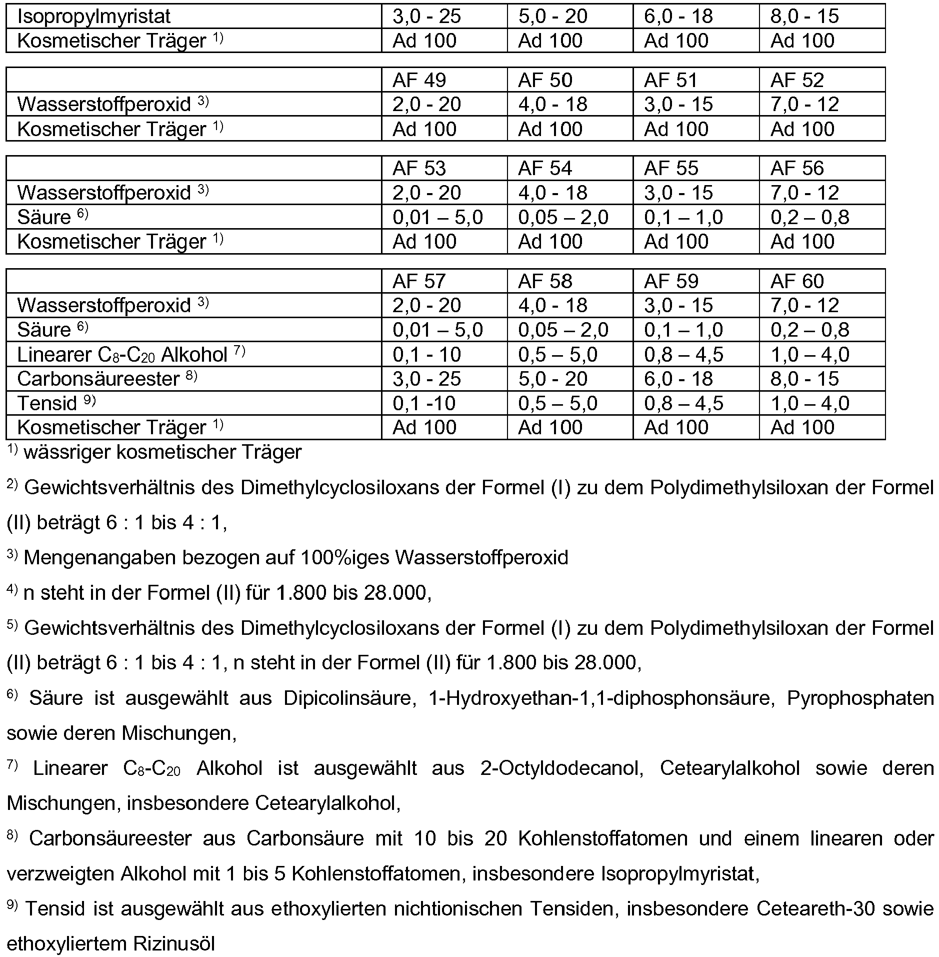

In bevorzugten erfindungsgemäßen Verfahren zur Aufhellung wird in Verfahrensschritt a) eine der Ausführungsformen 1 bis 28 des kosmetischen Mittels (M1) mit einer der Ausführungsformen AF 49 bis AF 60 der Oxidationsmittelzubereitung (M2) im Verhältnis 3 : 1 bis 1 : 3 vermischt. Besonders bevorzugt ist die Vermischung einer der Ausführungsformen 21 bis 28 des kosmetischen Mittels (M1) mit einer der Ausführungsformen 49 bis 60 der Oxidationsmittelzubereitung (M2) im Verhältnis 1 : 2.

Der Einsatz eines Aufhellmittels (AM), welches durch Vermischen einer der Ausführungsformen 1 bis 28 des kosmetischen Mittels (M1) mit einer der Ausführungsformen 29 bis 60 der Oxidationsmittelzubereitung (M2) erhältlich ist, in erfindungsgemäßen Aufhellverfahren führt zu einer verbesserten Pflege sowie einer verbesserten Aufhellleistung im Vergleich zu Aufhellmitteln, welche keine Siloxane der Formeln (I) bzw. (la) und (II) enthalten.

Besonders bevorzugte erfindungsgemäße Verfahren sind daher dadurch gekennzeichnet, dass die Verfahren in einer verbesserten Pflege der keratinischen Fasern bei gleichzeitig verbesserter Aufhellleistung resultieren. Durch die Verwendung einer Kombination von speziellen Siloxanen der Formeln (I) bzw. (la) und (II) ist die aus dem erfindungsgemäßen Verfahren resultierende Pflege und Aufhellleistung größer als die Pflege und Aufhellleistung, welche in Abwesenheit der speziellen Siloxane erreicht werden kann.

Erfindungsgemäß verwendete Aufhellmittel (AM) werden direkt vor der Anwendung aus zwei oder mehreren getrennt verpackten Zusammensetzungen hergestellt. Dies bietet sich insbesondere zur Trennung inkompatibler Inhaltsstoffe an, um eine vorzeitige Reaktion zu vermeiden. Das oxidative Aufhellmittel (AM) wird gemäß Verfahrensschritt a) des erfindungsgemäßen Verfahrens vom Anwender direkt vor der Verwendung durch Vermischen der Komponenten hergestellt. Es ist daher erfindungsgemäß vorgesehen das kosmetische Mittel (M1) zunächst getrennt von der Oxidationsmittelzubereitung (M2) zu konfektionieren.

Ein weiterer Gegenstand der vorliegenden Erfindung ist daher eine Verpackungseinheit (Kit-of-Parts), umfassend - getrennt voneinander konfektioniert -
a) mindestens einen Container (C1), enthaltend ein kosmetisches Mittel (M1), und
b) mindestens einen Container (C2), enthaltend eine Oxidationsmittelzubereitung (M2), welche mindestens ein Oxidationsmittel enthält,
wobei das kosmetische Mittel (M1) in dem Container (C1) und/oder die Oxidationsmittelzubereitung (M2) in dem Container (C2)
(i) mindestens ein Dimethylcyclosiloxan der Formel (I) 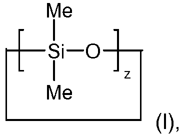 worin
   z für ganze Zahlen von 3 bis 12 steht, und
(ii) mindestens ein Polydimethylsiloxan der Formel (II) 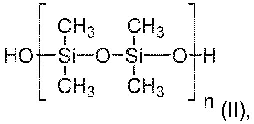 worin n für ganze Zahlen von 1.800 bis 28.000 steht,
enthält.

Unter dem Begriff "Container" wird im Rahmen der vorliegenden Erfindung eine Umhüllung verstanden, welche in Form einer gegebenenfalls wieder verschließbaren Flasche, einer Tube, einer Dose, eines Tütchens, eines Sachets oder ähnlichen Umhüllungen vorliegt. Dem Umhüllungsmaterial sind erfindungsgemäß keine Grenzen gesetzt. Bevorzugt handelt es sich dabei jedoch um Umhüllungen aus Glas oder Kunststoff.

Zur Herstellung des in Verfahrensschritt a) eingesetzten oxidativen Aufhellmittels (AM) aus der erfindungsgemäßen Verpackungseinheit (Kit-of-parts) wird das kosmetische Mittel (M1) in dem Container (C1) mit der Oxidationsmittelzubereitung (M2) in dem Container (C2) oder *vice versa* vermischt.

Weiterhin kann es erfindungsgemäß besonders vorteilhaft sein, wenn die Verpackungseinheit mindestens ein weiteres Haarbehandlungsmittel (M3), insbesondere eine Konditioniermittelzubereitung, in einem zusätzlichen Container (C3) enthält. Diese Konditioniermittelzubereitung enthält vorteilhafterweise mindestens ein Konditioniermittel, ausgewählt aus der Gruppe von kationischen Polymeren, Siliconderivaten und Ölen. Darüber hinaus kann die Verpackungseinheit Applikationshilfen, wie Kämme, Bürsten, Applicetten oder Pinsel, persönliche Schutzkleidung, insbesondere Ein-Weg-Handschuhe, sowie gegebenenfalls eine Gebrauchsanleitung umfassen. Unter einer Applicette wird ein breiter Pinsel verstanden, an dessen Stielende sich eine Spitze befindet, welche das Abteilen von Faserbündeln bzw. Strähnchen aus der Gesamtmenge der Fasern erlaubt und vereinfacht.

Bezüglich des Dimethylcyclosiloxans der Formel (I), des Polydimethylsiloxans der Formel (II) sowie der weiteren Wirk- und Inhaltsstoffe des kosmetischen Mittels (M1) und der Oxidationsmittelzubereitung (M2) gilt mutatis mutandis das zu den in dem erfindungsgemäßen Verfahren verwendeten kosmetischen Mitteln (M1) und Oxidationsmittelzubereitungen (M2) Gesagte.

Schließlich ist ein weiterer Gegenstand der vorliegenden Erfindung die Verwendung einer Kombination von
(i) mindestens ein Dimethylcyclosiloxan der Formel (I) 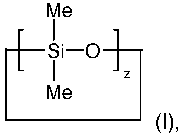 worin
   z für ganze Zahlen von 3 bis 12 steht, und
(ii) mindestens ein Polydimethylsiloxan der Formel (II) 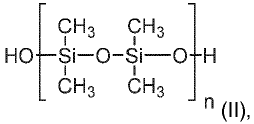 worin n für ganze Zahlen von 1.800 bis 28.000 steht,
zur Erhöhung der Pflege keratinischer Fasern bei gleichzeitig verbesserter Aufhellleistung. Unter dem Begriff der "Kombination" im Sinne der vorliegenden Erfindung wird eine Mischung des Dimethylcyclosiloxans (i) und des Polydimethylsiloxans (ii) verstanden. Der Einsatz der zuvor genannten Kombination von speziellen Siloxanen resultiert in einer erhöhten Pflege gefärbter keratinischer Fasern bei gleichzeitig verbesserter Aufhellleistung.

Bezüglich weiterer bevorzugter Ausführungsformen der erfindungsgemäßen Verwendung gilt mutatis mutandis das zu den in dem erfindungsgemäßen Verfahren verwendeten kosmetischen Mitteln (M1) und Oxidationsmittelzubereitungen (M2) sowie zu der erfindungsgemäßen Verpackungseinheit Gesagte. Die nachfolgenden Beispiele sollen bevorzugte Ausführungsformen der Erfindung erläutern, ohne sie jedoch einzuschränken.

### Beispiele:

### 1. Rezepturen

Zusammensetzungen der eingesetzten kosmetischen Mittel (M1) (ÖI-in-Wasser-Emulsionen, alle Mengen in Gew.-%). Das in den nachfolgenden Formulierungen verwendete Dimethylcyclosiloxan der Formel (I) ist bevorzugt ein Dimethylcyclosiloxan der Formel (la). Das bevorzugt eingesetzte Polydimethylsiloxan weist die Formel (II) mit n = 1.800 bis 28.000 auf. Besonders bevorzugt beträgt das Gewichtsverhältnis von Dimethylcyclosiloxan der Formel (I) und/oder (la) zu Polydimethylsiloxan der Formel (II) von 6 : 1 bis 4 : 1.

| Rohstoff | V1 | E1* |
|---|---|---|
| Xanthan Gum | 0,1 | 0,1 |
| 2-Octyldodecanol | 2,3 | 2,3 |
| Lanette N ^{a)} | 14 | 14 |
| Cetearyl Alkohol | 3,9 | 3,9 |
| Glycerinmonostearat | 6,0 | 6,0 |
| Glycerol 99,5 % | 2,0 | 2,0 |
| Kokosamidopropylbeatin, 40%ig | 2,0 | 2,0 |
| Monoethanolamin | 6,1 | 6,1 |
| 2-Amino-2-methylpropanol | 0,1 | 0,1 |
| Natriumsulfit, wasserfrei | 0,1 | 0,1 |
| Caramelsirup, 75%ig | 0,1 | 0,1 |
| Traubenkernöl | 1,0 | 1,0 |
| p-Toluylendiaminsulfat | 0,1 | 0,1 |
| Resorcinol | 0,04 | 0,04 |
| m-Aminophenol | 0,01 | 0,01 |
| 2,4-Diaminophenoxyethanol*2HCI | 0,01 | 0,01 |
| Dimethylcyclosiloxan der Formel (I) | -- | 1,7 |
| Polydimethylsiloxan der Formel (II) | -- | 0,3 |
| Wasser, vollentsalzt | ad 100,00 | ad 100,00 |

| | | |
|---|---|---|
| * erfindungsgemäß ^{a)} INCI-Bezeichnung: Cetearyl alcohol, Sodium cetearyl sulfate (BASF) | | |

Die Fettbasis wurde jeweils zusammen bei 80°C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die jeweilige Formulierung kalt gerührt.

**Oxidationsmittelzubereitung O1 (alle Mengen in Gew.-%)**

| Rohstoff | O1 |
|---|---|
| Dinatriumpyrophosphat | 0,1 |
| Dipicolinsäure | 0,1 |
| Kaliumhydroxid 50% | 0,3 |
| 1-Hydroxyethan-1,1-diphosphonsäure 60% | 0,4 |
| Natriumfettalkoholsulfat C₁₆-C₁₈ | 0,3 |
| Eumulgin RO 40 ^{b)} | 0,6 |
| Cetearylalkohol | 3,6 |
| Ceteareth-20 | 0,5 |
| Bienenwachs | 0,3 |
| Isopropylmyristat | 10 |
| Wasserstoffperoxid 50 % | 23 |
| Wasser vollentsalzt | ad 100 |

| | |
|---|---|
| ^{b)} INCI-Bezeichnung: PEG-40 Castor oil (BASF) | |

### 2. Verbesserte Pflege bei Verwendung von speziellen Siloxanen in dem erfindungsgemäßen Verfahren

12 Strähnen von natürlich hellbraunem europäischen Haar (IHIP (New York), lot# 03/2012, N121, lenght 15 cm, weight 1 g) wurden mit einer wässrigen Natrium-Laurylethersulfat-Lösung (3 % Aktivsubstanzgehalt in der Lösung) gewaschen. Die Strähnen wurden an der Luft getrocknet und für 24 h bei 25°C und 25% relativer Luftfeuchtigkeit gelagert. Nach Einweichen dieser Strähnen für 5 Minuten in Wasser wurde deren Nasskämmbarkeit bestimmt (Referenzwert).

Zur Herstellung der Aufhellmittel (AM-V1) und (AM-E1) wurde das jeweilige kosmetische Mittel V1 und E1 jeweils im Gewichtsverhältnis 1:2 mit der obigen Oxidationsmittelzubereitung O1 vermischt. Die zuvor herstellten Aufhellmittel (AM-V1) (nicht erfindungsgemäß) und (AM-E1) (erfindungsgemäß) wurden jeweils auf 12 Strähnen von natürlich europäischen Haar (IHIP (New York), lot # 03/2012, N121, lenght 15 cm, weight 1 g) appliziert, wobei jeweils 4 g des jeweiligen Aufhellmittels pro 1 g Haarsträhne verwendet wurde. Nachdem die Strähnen für 30 min bei 32 °C aufgehellt wurden, wurden sie für 2 min mit Wasser gespült und an der Luft getrocknet.

### Die Messung der Nasskämmbarkeit wurde wie folgt durchgeführt:

Vor der Messung wurde jede Strähne unter Kämmen mit einem harten Gummikamm mit feinen Zähnen (Firma Hercules Sägemann, Hamburg Germany) für 2 Sekunden mit Wasser befeuchtet. Nachdem 3 Kämmvorgänge durchgeführt wurden, wird die Kämmkraft während weiteren 10 Kämmvorgängen gemessen, wobei die jeweilige Haarsträhne während des Kämmvorgangs langsam rotiert. Die erhaltenen Messwerte werden unter Verwendung der folgenden in der Software Statistica 10.0 (StatSoft Inc., USA) eingebetteten statistischen Tests verglichen:
- Shapiro-Wilks Test (Test für Normalverteilung)
- Ausreißertest nach Grubbs
- Bartlett-Test (Test auf Homoskedastizität von Varianzen)
- Univarianter Signifikanztest
- Newman-Keuls Test (Bestimmung von signifikanten Unterschieden)
- Unequal N HSD Test (Test auf Mehrfachvergleiche).

Die Änderung der Kämmkraft dK in Prozent kann mit Hilfe der Formel dK = [(K₀-Kᵢ)/K₀]*100 berechnet werden. Ko ist hierbei der Mittelwert der Kämmkraft für die ungefärbten Haarsträhnen und Kᵢ der Mittelwert für die mit dem jeweiligen oxidativen Aufhellmitteln behandelten Haarsträhnen.

Die Pflege der Haarsträhnen ist umso höher, je geringer die aufgewendete Kämmkraft und damit je höher die Änderung der Kämmkraft ist. In nachfolgender Tabelle sind die dK-Werte für die Aufhellungen unter Verwendung der Aufhellmittel (AM-V1 = nicht erfindungsgemäß) und (AM-E1 = erfindungsgemäß) dargestellt. Die Aufhellungen, welche unter Verwendung von Aufhellmitteln mit speziellen Siloxanen (AM-E1) enthalten werden, weisen im Vergleich zu den Aufhellungen bei Einsatz von Aufhellmitteln ohne spezielle Siloxane (AM-V1) eine höhere Änderung der Kämmkraft und somit eine erhöhte Pflege auf.

| Aufhellmittel | dK [%] |
|---|---|
| AM-V1 (nicht erfindungsgemäß) | 33 |
| AM-E1 (erfindungsgemäß | 44 |

### 3. Verbesserte Aufhellleistung bei Verwendung von speziellen Siloxanen in dem erfindungsgemäßen Verfahren

Zur Herstellung der Aufhellmittel (AM-V1) und (AM-E1) wurde das jeweilige kosmetische Mittel V1 und E1 jeweils im Gewichtsverhältnis 1:2 mit der obigen Oxidationsmittelzubereitung O1 vermischt. Die zuvor herstellten Aufhellmittel (AM-V1) (nicht erfindungsgemäß) und (AM-E1) (erfindungsgemäß) wurden jeweils auf Strähnen von dunkelblonden, hellbraunen und dunkelbraunen Haar (Codes: Kerling 6/0, Fischbach & Miller 6923) von ca. 0,7 g Gewicht appliziert, wobei jeweils die 4-fache Menge des jeweiligen Aufhellmittels bezogen auf das Gewicht der Haarsträhne verwendet wurde. Nachdem die Strähnen für 45 min bei 32 °C aufgehellt wurden, wurden sie mit einem handelsüblichen Shampoo gewaschen und mit einem Föhn getrocknet.

Alle Strähnen wurden mit einem Farbmessgerät der Firma Datacolor, Typ Spectraflash 450, vermessen. Der für die Beurteilung der Aufhellleistung herangezogene Wert dL ergibt sich aus den an der jeweiligen Strähne gemessenen L*a*b-Farbmesswerten wie folgt: dL = Lᵢ-L₀
Lo ist hierbei jeweils der Mittelwert der aus den 12 Messungen ermittelten Farbmesswerte der unbehandelten Haarsträhnen, während Lᵢ jeweils den Mittelwert der Farbmesswerte nach der Aufhellung der Haarsträhnen mit dem jeweiligen oxidativen Aufhellmittel (AM-V1) bzw. (AM-E1) darstellt.

Je höher der dL-Wert ist, desto höher ist die mit dem jeweiligen Aufhellverfahren erreichte Aufhellleistung. In der nachfolgenden Tabelle sind die dL-Werte für die Aufhellungen unter Verwendung der Aufhellmittel (AM-V1) und (AM-E1) dargestellt. Die Aufhellungen, welche unter Verwendung von Aufhellmitteln mit speziellen Siloxanen (AM-E1) enthalten werden, weisen im Vergleich zu den Aufhellungen bei Einsatz von Aufhellmitteln ohne spezielle Siloxane (AM-V1) eine verbesserte Aufhellleistung auf.

| Aufhellmittel | dL |
|---|---|
| AM-V1 (nicht erfindungsgemäß) | 5,40 |
| AM-E1 (erfindungsgemäß) | 7,15 |

## Patentansprüche

1. Verfahren zur Aufhellung von keratinischen Fasern, wobei das Verfahren die folgenden Verfahrensschritte in der angegebenen Reihenfolge umfasst:
a) Auftragen eines Aufhellmittels (AM), welches unmittelbar vor der Auftragung aus mindestens einem kosmetischen Mittel (M1) und mindestens einer Oxidationsmittelzubereitung (M2) hergestellt wird, auf die keratinischen Fasern,
b) Belassen des unter Schritt a) hergestellten Aufhellmittels (AM) für eine Zeit von 10 bis 60 Minuten, bevorzugt von 20 bis 45 Minuten, bei Raumtemperatur und/oder bei mindestens 45 °C auf den keratinischen Fasern,
c) Spülen der keratinischen Fasern mit Wasser und/oder einer reinigenden Zusammensetzung für 1 bis 5 Minuten,
wobei das kosmetische Mittel (M1) und/oder die Oxidationsmittelzubereitung (M2) in einem kosmetisch verträglichen Träger
a. mindestens ein Dimethylcyclosiloxan der Formel (I) in einer Gesamtmenge von 1,2 bis 4,5 Gew.-%, bezogen auf das Gesamtgewicht des kosmetischen Mittels (M1) und/oder der Oxidationsmittelzubereitung (M2) 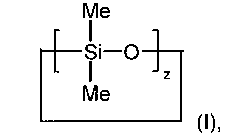 worin
z für ganze Zahlen von 3 bis 12 steht, und
b. mindestens ein Polydimethylsiloxan der Formel (II) in einer Gesamtmenge von 0,2 bis 1,0 Gew.-%, bezogen auf das Gesamtgewicht des kosmetischen Mittels (M1) und/oder der Oxidationsmittelzubereitung (M2) 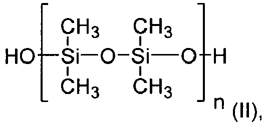 worin n für ganze Zahlen von 1.800 bis 28.000 steht, enthält, und
wobei das Gewichtsverhältnis des mindestens einen Dimethylcyclosiloxans (i) der Formel (I) zu dem mindestens einen Polydimethylsiloxan (ii) der Formel (II) in dem kosmetischen Mittel (M1) und/oder der Oxidationsmittelzubereitung (M2) 6 : 1 bis 4 : 1, beträgt.

2. Verfahren nach Anspruch 1, **dadurch gekennzeichnet, dass** in Verfahrensschritt a) das kosmetische Mittel (M1) mit der Oxidationsmittelzubereitung (M2) im Gewichtsverhältnis 3 : 1 bis 1 : 3, vorzugsweise von 2 : 1 bis 1 : 2, insbesondere 1 : 2, vermischt wird.

3. Verfahren nach einem der Ansprüche 1 oder 2, **dadurch gekennzeichnet, dass** das mindestens eine Dimethylcyclosiloxan (i) in dem kosmetischen Mittel (M1) und/oder in der Oxidationsmittelzubereitung (M2) die Formel (la) 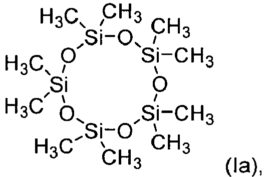 aufweist.

4. Verfahren nach einem der vorangehenden Ansprüche, **dadurch gekennzeichnet, dass** das mindestens eine Polydimethylsiloxan (ii) der Formel (II) in dem kosmetischen Mittel (M1) und/oder die Oxidationsmittelzubereitung (M2) eine Viskosität bei 25 °C von 100.000 bis 100.000.000 mPa*s, vorzugsweise von 200.000 bis 100.000.000 mPa*s, bevorzugt von 300.000 bis 100.000.000 mPa*s, insbesondere von 400.000 bis 100.000.000 mPa*s, aufweist.

5. Verfahren nach einem der vorangehenden Ansprüche, **dadurch gekennzeichnet, dass** das kosmetische Mittel (M1) und/oder die Oxidationsmittelzubereitung (M2)
a. mindestens ein Dimethylcyclosiloxan der Formel (Ia) 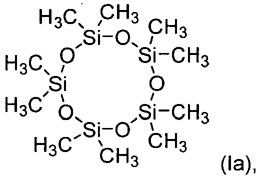 und
b. mindestens ein Polydimethylsiloxan der Formel (II) 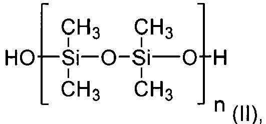 worin
n für 1.800 bis 28.000 steht, enthält.

6. Verfahren nach einem der vorangehenden Ansprüche, **dadurch gekennzeichnet, dass** das kosmetische Mittel (M1) und/oder die Oxidationsmittelzubereitung (M2) - bezogen auf das Gesamtgewicht des kosmetischen Mittels (M1) und/oder der Oxidationsmittelzubereitung (M2)
a. mindestens ein Dimethylcyclosiloxan der Formel (la) in einer Gesamtmenge von 1,2 bis 4,5 Gew.-% 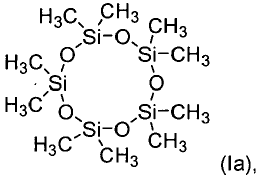
b. mindestens ein Polydimethylsiloxan der Formel (II) in einer Gesamtmenge von 0,2 bis 1,0 Gew.-% 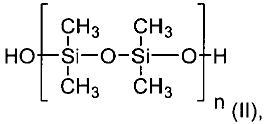 worin
n für 1.800 bis 28.000 steht, enthält,
wobei das Gewichtsverhältnis des mindestens einen Dimethylcyclosiloxans (i) der Formel (Ia) zu dem mindestens einen Polydimethylsiloxan (ii) der Formel (II) in dem kosmetischen Mittel (M1) und/oder der Oxidationsmittelzubereitung (M2) von 6 : 1 bis 4 : 1 beträgt.

7. Verfahren nach einem der vorangehenden Ansprüche, **dadurch gekennzeichnet, dass** das kosmetische Mittel (M1) zusätzlich mindestens eine farbgebende Verbindung, ausgewählt aus der Gruppe von Oxidationsfarbstoffvorprodukten, direktziehenden Farbstoffen sowie deren Mischungen, enthält.

8. Verfahren nach einem der vorangehenden Ansprüche, **dadurch gekennzeichnet, dass** die Oxidationsmittelzubereitung (M2) mindestens ein Oxidationsmittel aus der Gruppe von Wasserstoffperoxid und dessen Anlagerungsprodukten an Harnstoff, Melamin und Natriumborat, insbesondere Wasserstoffperoxid, in einer Gesamtmenge von 2,0 bis 20 Gew.-%, vorzugsweise von 4,0 bis 18 Gew.-%, bevorzugt von 3,0 bis 15 Gew.-%, insbesondere von 7,0 bis 12 Gew.-%, bezogen auf das Gesamtgewicht der Oxidationsmittelzubereitung (M2), enthält.

9. Verfahren nach einem der vorangehenden Ansprüche, **dadurch gekennzeichnet**, das Verfahren in einer verbesserten Pflege der keratinischen Fasern bei gleichzeitig verbesserter Aufhellleistung resultiert.

10. Verpackungseinheit (Kit-of-Parts), umfassend - getrennt voneinander konfektioniert -
a) mindestens einen Container (C1), enthaltend ein kosmetisches Mittel (M1), und
b) mindestens einen Container (C2), enthaltend eine Oxidationsmittelzubereitung (M2), welche mindestens ein Oxidationsmittel enthält,
wobei das kosmetische Mittel (M1) in dem Container (C1) und/oder die Oxidationsmittelzubereitung (M2) in dem Container (C2)
a. mindestens ein Dimethylcyclosiloxan der Formel (I) in einer Gesamtmenge von 1,2 bis 4,5 Gew.-%, bezogen auf das Gesamtgewicht des kosmetischen Mittels (M1) und/oder der Oxidationsmittelzubereitung (M2) 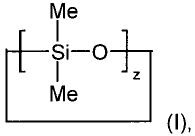 worin
z für ganze Zahlen von 3 bis 12 steht, und
b. mindestens ein Polydimethylsiloxan der Formel (II) in einer Gesamtmenge von 0,2 bis 1,0 Gew.-%, bezogen auf das Gesamtgewicht des kosmetischen Mittels (M1) und/oder der Oxidationsmittelzubereitung (M2) 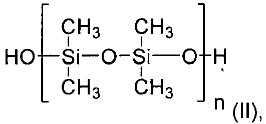 worin n für ganze Zahlen von 1.800 bis 28.000 steht,
enthält.

## Claims

1. A method for lightening keratin fibers, wherein the method comprises the following method steps in the sequence indicated:
a) applying a brightening agent (AM) to the keratin fibers, which brightening agent is prepared immediately before application from at least one cosmetic agent (M1) and at least one oxidizing agent preparation (M2),
b) leaving the brightening agent (AM) prepared in step a) on the keratin fibers for a time of from 10 to 60 minutes, preferably from 20 to 45 minutes, at room temperature and/or at least 45 °C,
c) rinsing the keratin fibers with water and/or a cleaning composition for 1 to 5 minutes, wherein the cosmetic agent (M1) and/or the oxidizing agent preparation (M2) contains, in a cosmetically acceptable carrier,
a. at least one dimethylcyclosiloxane of formula (I) in a total amount of from 1.2 to 4.5 wt.%, based on the total weight of the cosmetic agent (M1) and/or the oxidizing agent preparation (M2) 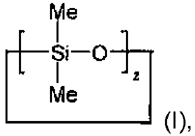 in which
z represents integers from 3 to 12, and
b. at least one polydimethylsiloxane of formula (II) in a total amount of from 0.2 to 1.0 wt.%, based on the total weight of the cosmetic agent (M1) and/or the oxidizing agent preparation (M2) 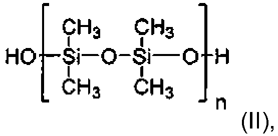 in which n represents integers from 1,800 to 28,000, and
wherein the weight ratio of the at least one dimethylcyclosiloxane (i) of formula (I) to the at least one polydimethylsiloxane (ii) of formula (II) in the cosmetic agent (M1) and/or the oxidizing agent preparation (M2) is 6:1 to 4:1.

2. The method according to claim 1, **characterized in that**, in method step a), the cosmetic agent (M1) is mixed with the oxidizing agent preparation (M2) in a weight ratio of 3:1 to 1:3, preferably from 2:1 to 1:2, in particular 1:2.

3. The method according to one of claims 1 or 2, **characterized in that** the at least one dimethylcyclosiloxane (i) in the cosmetic agent (M1) and/or in the oxidizing agent preparation (M2) has the formula (Ia)

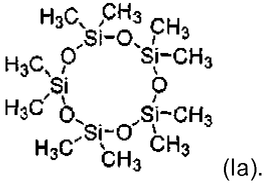

4. The method according to one of the preceding claims, **characterized in that** the at least one polydimethylsiloxane (ii) of formula (II) in the cosmetic agent (M1) and/or the oxidizing agent preparation (M2) has a viscosity at 25 °C of from 100,000 to 100,000,000 mPa·s, preferably from 200,000 to 100,000,000 mPa·s, more preferably from 300,000 to 100,000,000 mPa·s, in particular from 400,000 to 100,000,000 mPa·s.

5. The method according to one of the preceding claims, **characterized in that** the cosmetic agent (M1) and/or the oxidizing agent preparation (M2) contains
a. at least one dimethylcyclosiloxane of formula (Ia) 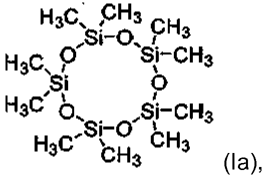 and
b. at least one polydimethylsiloxane of formula (II) 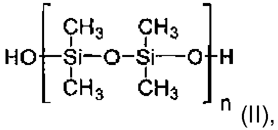 in which
n represents 1,800 to 28,000.

6. The method according to one of the preceding claims, **characterized in that** the cosmetic agent (M1) and/or the oxidizing agent preparation (M2) contains, based on the total weight of the cosmetic agent (M1) and/or the oxidizing agent preparation (M2),
a. at least one dimethylcyclosiloxane of formula (Ia) in a total amount of from 1.2 to 4.5 wt.% 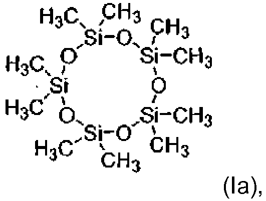
b. at least one polydimethylsiloxane of formula (II) in a total amount of from 0.2 to 1.0 wt.% 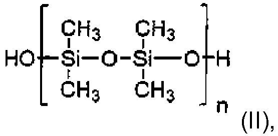 in which
n represents 1,800 to 28,000,
the weight ratio of the at least one dimethylcyclosiloxane (i) of formula (Ia) to the at least one polydimethylsiloxane (ii) of formula (II) in the cosmetic agent (M1) and/or the oxidizing agent preparation (M2) being from 6:1 to 4:1.

7. The method according to one of the preceding claims, **characterized in that** the cosmetic agent (M1) additionally contains at least one chromophoric compound selected from the group of oxidation dye precursors, direct dyes and mixtures thereof.

8. The method according to one of the preceding claims, **characterized in that** the oxidizing agent preparation (M2) contains at least one oxidizing agent from the group of hydrogen peroxide and its addition products of urea, melamine and sodium borate, in particular hydrogen peroxide, in a total amount of from 2.0 to 20 wt.%, preferably from 4.0 to 18 wt.%, more preferably from 3.0 to 15 wt.%, in particular from 7.0 to 12 wt.%, based on the total weight of the oxidizing agent preparation (M2).

9. The method according to one of the preceding claims, **characterized in that** the method results in improved care of the keratin fibers with simultaneously improved brightening performance.

10. A packaging unit (kit-of-parts) comprising, packaged separately from one another,
a) at least one container (C1) containing a cosmetic agent (M1), and
b) at least one container (C2) containing an oxidizing agent preparation (M2) which contains at least one oxidizing agent,
wherein the cosmetic agent (M1) in the container (C1) and/or the oxidizing agent preparation (M2) in the container (C2) contains
a. at least one dimethylcyclosiloxane of formula (I) in a total amount of from 1.2 to 4.5 wt.%, based on the total weight of the cosmetic agent (M1) and/or the oxidizing agent preparation (M2) 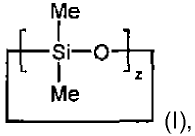 in which
z represents integers from 3 to 12, and
b. at least one polydimethylsiloxane of formula (II) in a total amount of from 0.2 to 1.0 wt.%, based on the total weight of the cosmetic agent (M1) and/or the oxidizing agent preparation (M2) 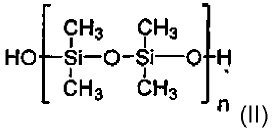 in which n represents integers from 1,800 to 28,000.

## Revendications

1. Procédé d'éclaircissement de fibres kératiniques, le procédé comprenant les étapes suivantes, dans l'ordre indiqué :
a) l'application d'un agent de blanchiment (AM) sur les fibres kératiniques, ledit agent de blanchiment étant préparé, à partir d'au moins un agent cosmétique (M1) et d'au moins une préparation d'agent d'oxydation (M2), immédiatement avant l'application,
b) le maintien, sur les fibres kératiniques, de l'agent de blanchiment (AM) préparé à l'étape a) pendant une durée de 10 à 60 minutes, de préférence de 20 à 45 minutes à température ambiante et/ou à au moins 45 °C,
c) le rinçage des fibres kératiniques avec de l'eau et/ou une composition détergente pendant 1 à 5 minutes,
l'agent cosmétique (M1) et/ou la préparation d'agent d'oxydation (M2) contenant, dans un support cosmétiquement acceptable,
a. au moins un diméthylcyclosiloxane de formule (I) en une quantité totale de 1,2 à 4,5 % en poids, par rapport au poids total de l'agent cosmétique (M1) et/ou de la préparation d'agent d'oxydation (M2) 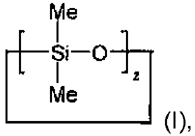 dans laquelle
z représente des nombres entiers compris entre 3 et 12, et
b. au moins un polydiméthylsiloxane de formule (II) en une quantité totale de 0,2 à 1,0 % en poids, par rapport au poids total de l'agent cosmétique (M1) et/ou de la préparation d'agent d'oxydation (M2) 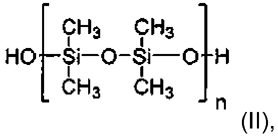 dans laquelle n représente des nombres entiers compris entre 1 800 et 28 000, et
le rapport pondéral de l'au moins un diméthylcyclosiloxane (i) de formule (I) à l'au moins un polydiméthylsiloxane (ii) de formule (II) dans l'agent cosmétique (M1) et/ou la préparation d'agent d'oxydation (M2) étant de 6:1 à 4:1.

2. Procédé selon la revendication 1, **caractérisé en ce que**, à l'étape a), l'agent cosmétique (M1) est mélangé à la préparation d'agent d'oxydation (M2) dans le rapport pondéral de 3:1 à 1:3, de préférence de 2:1 à 1:2, en particulier de 1:2.

3. Procédé selon l'une des revendications 1 ou 2, **caractérisé en ce que** l'au moins un diméthylcyclosiloxane (i) dans l'agent cosmétique (M1) et/ou dans la préparation d'agent d'oxydation (M2) présente la formule (Ia)

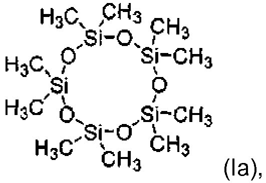

4. Procédé selon l'une des revendications précédentes, **caractérisé en ce que** l'au moins un polydiméthylsiloxane (ii) de formule (II) dans l'agent cosmétique (M1) et/ou la préparation d'agent d'oxydation (M2) présente une viscosité à 25 °C de 100 000 à 100 000 000 mPa*s, de préférence de 200 000 à 100 000 000 mPa*s, de manière préférée de 300 000 à 100 000 000 mPa*s, en particulier de 400 000 à 100 000 000 mPa*s.

5. Procédé selon l'une des revendications précédentes, **caractérisé en ce que** l'agent cosmétique (M1) et/ou la préparation d'agent d'oxydation (M2) contient
a. au moins un diméthylcyclosiloxane de formule (Ia) 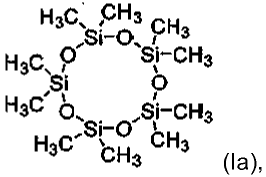 et
b. au moins un polydiméthylsiloxane de formule (II) 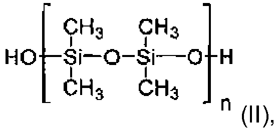 dans laquelle
n représente 1 800 à 28 000.

6. Procédé selon l'une des revendications précédentes, **caractérisé en ce que** l'agent cosmétique (M1) et/ou la préparation d'agent d'oxydation (M2) contient, par rapport au poids total de l'agent cosmétique (M1) et/ou de la préparation d'agent d'oxydation (M2),
a. au moins un diméthylcyclosiloxane de formule (la) en une quantité totale de 1,2 à 4,5 % en poids 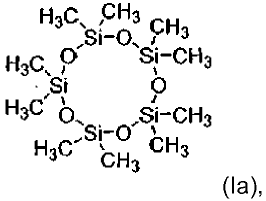
b. au moins un polydiméthylsiloxane de formule (II) en une quantité totale de 0,2 à 1,0 % en poids 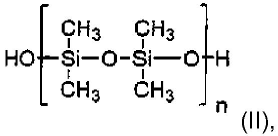 dans laquelle
n représente 1 800 à 28 000,
le rapport pondéral de l'au moins un diméthylcyclosiloxane (i) de formule (la) à l'au moins un polydiméthylsiloxane (ii) de formule (II) dans l'agent cosmétique (M1) et/ la préparation d'agent d'oxydation (M2) est de 6:1 à 4:1.

7. Procédé selon l'une des revendications précédentes, **caractérisé en ce que** l'agent cosmétique (M1) contient en outre au moins un composé colorant choisi dans le groupe constitué par des précurseurs de colorants d'oxydation, des colorants directs ainsi que leurs mélanges.

8. Procédé selon l'une des revendications précédentes, **caractérisé en ce que** la préparation d'agent d'oxydation (M2) contient au moins un agent d'oxydation choisi dans le groupe constitué par le peroxyde d'hydrogène et ses produits d'addition d'urée, de mélamine et de borate de sodium, en particulier le peroxyde d'hydrogène, en une quantité totale de 2,0 à 20 % en poids, de préférence de 4,0 à 18 % en poids, de manière préférée de 3,0 à 15 % en poids, en particulier de 7,0 à 12 % en poids, par rapport au poids total de la préparation d'agent d'oxydation (M2).

9. Procédé selon l'une des revendications précédentes, **caractérisé en ce que** le procédé permet de mieux soigner les fibres kératiniques tout en améliorant le pouvoir éclaircissant.

10. Unité de conditionnement (Kits-of-Parts) comprenant, confectionnés séparément les uns des autres,
a) au moins un récipient (C1) contenant un agent cosmétique (M1), et
b) au moins un récipient (C2) contenant une préparation d'agent d'oxydation (M2), contenant au moins un agent d'oxydation,
l'agent cosmétique (M1) dans le récipient (C1) et/ou la préparation d'agent d'oxydation (M2) dans le récipient (C2) contenant
a. au moins un diméthylcyclosiloxane de formule (I) en une quantité totale de 1,2 à 4,5 % en poids, par rapport au poids total de l'agent cosmétique (M1) et/ou de la préparation d'agent d'oxydation (M2) 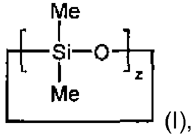 dans laquelle
z représente des nombres entiers compris entre 3 et 12, et
b. au moins un polydiméthylsiloxane de formule (II) en une quantité totale de 0,2 à 1,0 % en poids, par rapport au poids total de l'agent cosmétique (M1) et/ou de la préparation d'agent d'oxydation (M2) 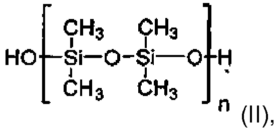 dans laquelle n représente des nombres entiers compris entre 1 800 et 28 000.
